# EUROPEAN PATENT SPECIFICATION

(11) **EP 1 995 307 B1**
(45) Date of publication and mention of the grant of the patent: **24.09.2014**
(21) Application number: 08012031.4
(22) Date of filing: 22.06.2006
(51) Int. Cl.: C12N 1/20, C12N 1/38, A61K 35/74, C12R 1/225

(54) **Methods and means for protecting the skin against pathogenic microorganisms**
Verfahren und Mittel zum Schutz der Haut vor krankheitserregenden Mikroorganismen
Procédés et supports de protection de la peau contre des micro-organismes pathogènes

(30) Priority: 22.06.2005 EP 05013494; 28.11.2005 US 740084 P
(43) Date of publication of application: 26.11.2008
(62) Divisional of application: 06754518.6
(73) Proprietor: OrganoBalance GmbH, 13355 Berlin (DE)
(72) Inventor: Lang, Christine, 10625 Berlin (DE); Heilman, Andreas, 12247 Berlin (DE); Veen, Markus, 84453 Altmühldorf (DE); Budde, Eckhard, 50733 Köln (DE); Boettner, Mewes, 10407 Berlin (DE); Reindl, Andreas, 68199 Mannheim (DE); Knöll, Rolf, 69514 Laudenbach (DE)
(74) Representative: Jungblut & Seuss

(56) References cited:
- WO-A-03/080813
- WO-A2-00/71139
- WO-A2-99/07332
- FALSEN E ET AL: "PHENOTYPIC AND PHYLOGENETIC CHARACTERIZATION OF A NOVEL LACTOBACILLUS SPECIES FROM HUMAN SOURCES: DESCRIPTION OF LACTOBACILLUS INERS SP. NOV" INTERNATIONAL JOURNAL OF SYSTEMATIC BACTERIOLOGY, SOCIETY FOR GENERAL MICROBIOLOGY, READING, GB, vol. 49, no. 1, January 1999 (1999-01), pages 217-221, XP008019383 ISSN: 0020-7713
- OCANA VIRGINIA S ET AL: "Growth inhibition of Staphylococcus aureus by H2O2-producing Lactobacillus paracasea subsp. paracasei isolated from the human vagina" FEMS IMMUNOLOGY AND MEDICAL MICROBIOLOGY, vol. 23, no. 2, February 1999 (1999-02), pages 87-92, XP002358245 ISSN: 0928-8244
- DATABASE BIOSIS [Online] BIOSCIENCES INFORMATION SERVICE, PHILADELPHIA, PA, US; 2003, CATALOLUK OSMAN ET AL: "Characterization of salivaricin B, a protein expressed by Lactobacillus salivarius M7." XP002358247 Database accession no. PREV200300522983 & TURKISH JOURNAL OF BIOLOGY, vol. 27, no. 3, 2003, pages 131-136, ISSN: 1300-0152
- KORTING H C ET AL: "INFLUENCE OF THE PH-VALUE ON THE GROWTH OF STAPHYLOCOCCUS-EPIDERMIDIS STAPHYLOCOCCUS-AUREUS AND PROPIONIBACTERIUM-ACNES IN CONTINUOUS CULTURE" ZENTRALBLATT FUR HYGIENE UND UMWELTMEDIZIN, vol. 193, no. 1, 1992, pages 78-90, XP008057020 ISSN: 0934-8859
- ROTH R R ET AL: "MICROBIAL ECOLOGY OF THE SKIN" ORNSTON, L. N. (ED.). ANNUAL REVIEW OF MICROBIOLOGY, VOL. 42. XII+794P. ANNUAL REVIEWS, INC.: PALO ALTO, CALIFORNIA, USA. ILLUS SERIES : ANNUAL REVIEW OF MICROBIOLOGY (ISSN 0066-4227), 1988, pages 441-464, XP008057017 ISSN: 0-8243-1142-6

## Description

The present invention relates to microorganisms which do not inhibit the growth of microorganisms of the healthy normal resident skin microbial flora and which inhibit the growth of microorganisms of the transient pathogenic skin micro flora. The present invention also relates to compositions, comprising such microorganisms, e.g. cosmetical or pharmaceutical compositions and to the use of such microorganisms in cosmetic, prophylactic or therapeutic applications.

The human skin is populated by a large variety of microorganisms that mainly live as commensals in a relatively stable composition on the surface of the skin (Roth and James, 1988). This normal skin flora is termed "resident skin flora".

The main function of the human skin is to protect the tissue beneath it against the environment (Feingold, 1985). This normal skin flora especially protects the skin against the intrusion of potentially pathogenic microorganisms (Bisno, 1984). Certain microorganisms dominate the resident microbial flora. More than ninety percent of the microorganisms of the resident microbial flora are *Staphylococcus epidermidis* (coagulase negative), *Micrococcus* spec., Diphteroids and propionibacteria (Leyden et al., 1987). Therefore, a stabilisation of the natural skin flora supports the protection of the skin and prevents the intrusion of pathogens. The health of the skin increases. The importance of the natural skin flora has been described in several clinical studies. It has been shown that in the first days after birth of an infant, where this skin flora has not yet been developed, the danger of a *Staphylococcus aureus* infection is very high. With increasing development of the flora, the skin is protected from the colonization by pathogenic microorganisms (Hurst, 1959). In another study with infants, it has been observed that after treatment with the antibiotic amoxicillin, the resident flora was drastically (about 50%) repressed. This led to more than a fourteen-fold increase of the pathogenic yeast *Candida albicans*. The discontinuation of the antibiotic treatment led to a regeneration of the resident flora and the repression of *Candida albicans* (Brook, 2000).

The microorganisms of the resident skin flora prevent the colonization by pathogenic microorganisms by competing for attachment sites and essential nutrients on the skin surface (Sullivan et al. 2001). Pathogenic microorganisms are able to specifically attach to structures of the epidermis using special binding proteins. In this context, different mechanisms are known. From *Staphylococcus aureus,* for example, specific adhesins are known. These allow the pathogenic microorganism to attach to fibronectin structures. Pathogens generally have a higher potential to attach to the host. This explains the virulence of these microorganisms (Gibbons and Houte, 1975).

The danger of colonization by pathogenic microorganisms increases drastically in the case of small lesions or other damages on the surface of the skin, especially when the normal skin flora is damaged by antibiotics or by excessive washing (Elek, 1956). However, the resident skin flora is better adapted to the skin regarding nutrient utilisation. This leads to an advantage of the resident skin flora (Larson, 2001). Apart from this, the organisms of the resident skin flora are able to produce antimicrobial substances to fight against pathogenic microorganisms. This is also an advantage for resident microorganisms regarding nutrients and energy sources (Selwyn and Ellis, 1972; Milyani and Selwyn, 1978).

Moreover, substances that are secreted by the skin, like complex lipids (triglycerides), are degraded to unsaturated fatty acids that inhibit pathogenic microorganisms like *Streptococcus pyrogenes* or gram negative bacteria and fungi (Aly et al., 1972).

The microbial skin flora affects several factors of the skin that are of cosmetic relevance. These are pH value of the skin, barrier function and lipid content. S. *epidermidis* is able to fight against pathogenic microorganisms by lowering the pH value (about 4-6). Pathogens are not able to grow at decreased pH values (Korting et al., 1990; Lukas, 1990; Korting, 1992; Yosipovitch and Maibach, 1996; Gfatter et al., 1997).

The water barrier function and the lipid content of the skin depend on the ceramide content of the horny layers (Imokawa et al., 1986). Lowering of the ceramide content causes a drying and rifting of the skin. A study with atopical dermatitis patients having these appearances of the skin showed that the microbial skin flora dramatically changes to *Staphylococcus aureus.* This pathogen features a very high ceramidase activity, while normal commensals of the resident skin flora do not have this activity. Sphingomyelinase activities that lead to the release of ceramides in the skin are comparable in the resident and pathogenic flora of atopic dermatitis patients (Ohnishi et al., 1999).

The document WO 03/080813 A discloses a Lactobacillus paracasei strain which adheres to vaginal epitalial cells and forms a barrier to prevent colonisation of pathogenic bacteria and its use as a probiotic composition for treating vaginal infections. The document Ocana Virginia S et al: "Growth inhibition of Staphylococcus aureus by H2O2-producing Lactobacillus paracasea subsp. paracasei isolated from the human vagina" FMS IMMUNOLOGY AND MEDICAL MICROBIOLOGY, vol. 23, no. 2, February 1999 (1999-02), pages 87-92, ISSN: 0928-8244, discloses growth inhibition of Staphylococcus aureus by H₂O₂-producing L. paracasei subspecies paracasei. The document Falsen E et al: "Phenytypic and phylogenetic characterization of a novel lactobacillus iners sp. nov" International Journal of Systematic Bacteriology, Society for General Microbiology, Reading, GB, Vol. no. 49, no. 1, January 1999 (1999-01), pages 217-221, ISSN: 0020-7713, discloses Lactobacillus paracasei, Lactobacillus brevis and Lactobacillus fermentum strains.

Thus, there is a need for means and methods allowing to protect the skin, in particular the human skin, against pathogenic microorganisms.

The present invention addresses this need and provides microorganisms and methods which protect the skin against the colonization by pathogenic microorganisms as characterized in the claims.

The inventors surprisingly found that an effective protection of the skin against a colonization by pathogenic microorganisms can be achieved by administering to the skin the above described microorganisms or inactivated forms thereof. The inventors for the first time identified corresponding microorganisms and provided methods for their identification. These microorganisms are able to differentially suppress the growth of microorganisms of the skin, i.e. they selectively inhibit the growth of pathogenic microorganisms, but do not influence the growth of the inhabitants of the healthy commensal micro flora. Thereby these microorganisms are able to regenerate and to stabilize the natural skin flora.

Many different microorganisms exist on the skin. Some belong to the normal (resident) flora of the skin and are harmless commensals and some are potential pathogens.

Basically, organisms on the skin can be classified into two categories: 1. Resident organisms: resident organisms are permanent inhabitants of the skin which colonise on the surface of the skin, the stratum corneum and within the outer layer of the epidermis and the deeper crevices of the skin and hair follicles. These microorganisms of the resident microbial skin flora can grow and multiply on the skin without invading or damaging the skin tissue. Washing does not easily remove these organisms in deeper skin regions. Resident microorganisms are harmless commensals.

2. Transient organisms: transient organisms are microorganisms which are deposited on the skin but do not multiply there or contaminants which multiply on the skin and persist for short periods. They cannot settle permanently on healthy skin whose microenvironment is heavily determined by the resident skin micro flora. Transient organisms are potentially pathogenic.

Thus, the term "transient pathogenic skin micro flora" refers to microorganisms which are deposited on the skin but do not multiply there or to contaminants which multiply on the skin and persist for short periods. In particular, if a microorganism is applied to the skin and is unable to grow and reproduce there under the environmental conditions provided by the healthy skin and cannot permanently colonize this organ (or a region of it), it is considered to belong to the transient pathogenic skin micro flora. Several bacteria, yeast and fungi can be transiently isolated from human skin but particularly the following microorganism can be classified to the transient pathogenic skin micro flora due to their frequent appearance: *Staphylococcus aureus, Streptococcus pyogenes,* gram-negative bacilli (e.g *Acinetobacter calcoaceticus), Candida albicans* and *Malassezia furfur.* Microorganisms of the transient micro flora often have pathogenic factors that allow the bacterium to attach to disordered skin regions. This can e.g. be the attachment to collagen structures or keratin structures.

The constituents and the composition of the microbial skin flora can be determined quantitatively and qualitatively, e.g. by peeling off the upper skin layers with scotch tape. Microorganisms of the skin micro flora can be identified within the upper ten skin layers peeled off, e.g., by scotch tape. Exemplary, to isolate these microorganisms six 2 cm² scotch tapes are each pressed on a defined region of the skin, preferably of the forearm and afterwards each tape stripe is transferred from the skin to a selective culture agar plate for either gram positive (e.g. BHI, Difco Inc.) or gram negative bacteria (e.g. MacConkey agar, Difco Inc.) or to a selective culture agar for yeasts and fungi (e.g. Plate Count Agar, Difco Inc.). Afterwards the microorganisms that have been transferred from skin to culture agar plates are cultivated at 30°C and 37°C, aerobically and anaerobically for about 24 hours. Colony forming units are determined by morphological and biochemical methods for a qualitative analysis and by counting for quantification. The relative composition and total cell counts are determined. The person skilled in the art can determine the genus and/or species of the microorganisms of the skin micro flora which have been isolated as described above by methods known in the art.

The microorganisms of the transient pathogenic skin micro flora can be determined, e.g., by metabolic footprinting, the evaluation of fatty acid composition and the composition of the cell wall, sequencing of 16S ribosomal RNA or the detection of specific DNA probes encoding specific pathogenic factors.

A microorganism is regarded as inhibiting the growth of a microorganism of the transient pathogenic skin micro flora if it leads to a decrease of growth of such a microorganism of the transient pathogenic skin micro flora when contacted with it. The term "inhibits the growth of microorganisms of the transient pathogenic skin micro flora" means that the microorganism of the invention decreases the growth of at least one, preferably of more than one, preferably of more than two, more preferably of more than five and particularly preferred of any of the microorganisms of the transient pathogenic flora. In a further preferred embodiment, the microorganism of the present invention inhibits the growth of the major representative of the transient pathogenic skin micro flora, i.e. *Staphylococcus aureus.* In a further preferred embodiment, the microorganism of the present invention specifically inhibits the growth of *Staphylococcus aureus.* "Specifically" preferably means that it inhibits the growth of *Staphylococcus aureus,* but does not significantly or only to a minor degree inhibit the growth of other microorganisms, in particular of those microorganisms which belong to the resident skin micro flora. More preferably, the term "specifically" means that the degree of inhibition on Staphylococcus is much higher than the degree of inhibition on another microorganism, in particular a microorganism of the resident skin micro flora. Particularly preferred, the term "specifically" means that in a suitable growth assay known to the person skilled in the art the proliferation of *Staphylococcus aureus* in the presence of the microorganism of the present invention is at the most 50% of the proliferation of another microorganism, in particular another microorganism of the resident skin micro flora in the presence of the microorganism of the present invention. Preferably, the proliferation of *Staphylococcus aureus* is 40%, 30%, 20%, 10%, more preferably 5% and most preferably 0% of the proliferation of another microorganism, in particular another microorganism of the resident skin micro flora, in the presence of a microorganism of the present invention. The specific inhibition of Staphylococcus aureus is indicated in Examples 10 and 11, which show by way of illustration that Micrococcus luteus and Escherichia coli are not inhibited by a microorganism according to the present invention in an in vitro liquid assay. In a preferred embodiment the microorganism of the present invention inhibits the growth of Staphylococcus aureus but does not inhibit the growth of *Micrococcus luteus* and/or *Escherichia coli.*

In a particularly preferred embodiment the specific inhibition of Staphylococcus aureus can be detected when culture conditions are used which include glycerol.

A decreased growth means preferably a decrease in proliferation, i.e. in cell divisions per unit. Alternatively, the term "inhibits" also refers to a decrease in size of individual cells. Bacterial cell size can be assessed by flow cytometry (e.g. Becton-Dickinson FACSort flow cytometer, San José, CA) after staining with the stain SYBR Green I (Molecular Probes, USA). Bacteria cell size is assessed in Side-Angle Light Scatter (SSC) mode.

A decreased growth thus means a decrease in biomass production per time unit.

The inhibition of growth of the microorganism(s) of the transient pathogenic skin micro flora can preferably be observed in vitro, more preferably in an assay in which a microorganism according to the invention is contacted with one or more microorganisms of the transient pathogenic skin micro flora and the growth of the(se) microorganism(s) of the transient pathogenic skin micro flora is determined. The growth can be determined by counting the numbers of cells/colonies after different time intervals of incubation and can be compared with a control which does not contain a microorganism according to the invention, thereby allowing to determine whether there is an increase or decrease in growth.

An in vitro assay for determining the inhibition of growth is described in the Examples and comprises a so-called "in vitro hole plate assay". In brief, such an assay comprises the following steps:
- cultivation of at least one microorganism of the transient pathogenic skin micro flora and evenly spreading it/them on a prepared agar plate containing a suitable agar medium for growth, and preferably detection, of the respective microorganism(s);
- providing holes in the inoculated agar plate;
- filling the holes with precultured cells of a microorganism according to the invention;
- incubating the agar plates for an appropriate amount of time and under conditions allowing growth of the microorganism(s) of the transient pathogenic skin micro flora; and
- determining the growth of the microorganism(s) of the transient pathogenic skin micro flora surrounding the holes containing a microorganism according to the invention and comparing it to the growth of the microorganism(s) surrounding a hole which does not contain a microorganism according to the invention.

The determination of the growth in the last step may be effected by available means and methods for determining the number of cells and/or colonies, e.g. by staining with an appropriate dye and/or optical means such as densitometry and counting the cells/colonies under the microscope. In a preferred embodiment the diameter of the occurring clearing zone next to the hole may be used to determine the area of inhibition.

More preferably, the inhibition of growth of the microorganism(s) of the transient pathogenic skin micro flora can be determined in an "in vitro liquid assay". Such an assay is described in the Examples and, briefly, comprises the following steps:
- cultivation of at least one microorganism of the transient pathogenic skin micro flora in a liquid culture;
- applying an aliquot of a liquid culture of the microorganism according to the invention and an aliquot of a liquid culture of the microorganism of the transient pathogenic skin micro flora to a culture medium allowing the growth of the microorganism of the transient pathogenic skin micro flora;
- co-cultivation of the microorganism according to the invention and the microorganism of the transient pathogenic skin micro flora in a liquid culture;
- transferring an aliquot of the co-cultivation liquid culture to an agar plate, containing an appropriate growth medium;
- incubation of the agar plates for a period of time and under conditions allowing the growth of the microorganism(s) of the transient pathogenic skin micro flora;
- determining the growth of the microorganism(s) of the transient pathogenic skin micro flora by quantification of the colony forming units and comparing it to the growth of the microorganism(s) in a control in which no microorganism of the invention was applied.

Even more preferably, the inhibition of growth of the microorganism(s) of the transient pathogenic skin micro flora can also be observed in an "in situ skin assay". Such assay is described in the Examples and, in brief, comprises the following steps:
- cultivation of at least one microorganism of the transient pathogenic skin micro flora and evenly spreading it on an area of skin of a test individual;
- applying an aliquot of a microorganism according to the invention in a punctual area within the area on which the microorganism(s) of the transient pathogenic skin micro flora has/have been spread;
- incubating the skin for an amount of time sufficient to allow growth of the microorganism(s) of the transient pathogenic skin micro flora;
- transferring the upper skin layers, including the microorganisms comprised in these, to an agar plate containing an appropriate growth medium;
- incubation of the agar plates for a period of time and under conditions allowing the growth of the microorganism(s) of the transient pathogenic skin micro flora;
- determining the growth of the microorganism(s) of the transient pathogenic skin micro flora surrounding the area at which the microorganism according to the invention was applied and comparing it to the growth of the microorganism(s) in a control in which no microorganism of the invention was applied.

The area of skin used for this assay may be any suitable area of skin of an individual, preferably of a human individual. In a preferred embodiment it is an area of skin on the forearm of a human individual. The size of the area is not decisive, preferably it is about 1 to 40 cm², more preferably 5 to 20 cm², even more preferably 5 to 10 cm² , e.g. about 5,6, 7, 8, 9 or 10 cm².

The microorganism(s) of the transient pathogenic skin micro flora are evenly distributed on the area, preferably in a density of approximately 10² cfu/cm² - 10³ cfu/cm². The microorganism(s), spread on the skin are air dried and an aliquot of a microorganism according to the invention is applied in a punctual manner within the area. This can be achieved by means known to the person skilled in the art. For example, the microorganisms according to the invention are centrifuged (15 min, 4000 x g). The cell pellet is washed two times with K/Na-buffer (each 1 ml). Cells are resuspended in 200 µl K/Na buffer and 10 µl of prepared microorganisms are punctual applied on the pre-inoculated skin area with a micro pipet.

The incubation of the skin preferably takes place at room temperature for, e.g., two hours. The transfer of the upper skin layers, including the microorganisms comprised therein, may, e.g., be effected with the help of an adhesive tape stripe. The agar plates to which the upper skin layers have been transferred are incubated at a temperature allowing growth of the microorganism(s) or the transient pathogenic skin micro flora to be tested and contain a growth medium known to support growth of this (these) microorganism(s). The incubation typically takes place for about 24 hours. The growth of the microorganism(s) can be detected by methods known to the person skilled in the art. Preferably, it is determined by densitometry or by counting the colonies formed in the neighborhood of the point at which an aliquot of the microorganism of the invention was applied. Bacterial cell size can be assessed by flow cytometry (e.g. Becton-Dickinson FACSort flow cytometer, San José, CA) after staining with the stain SYBR Green I (Molecular Probes, USA). Bacteria cell size is assessed in Side-Angle Light Scatter (SSC) mode.

A microorganism is regarded to inhibit the growth of one or more microorganisms of the pathogenic transient micro flora if it leads to a decrease of growth of at least one such microorganism in an "in vitro hole plate assay" of at least 5 %, preferably of at least 10%, 20%, 30%, 40%, 50%, 60%, or 70%, 80%, more preferably of at least 90% and even more preferably of at least 95% and most preferably of at least 99% in comparison to a control to which no microorganism has been added.

More preferably, a microorganism is regarded to inhibit the growth of one or more microorganisms of the pathogenic transient micro flora if it leads to a decrease of growth of at least one such microorganism in an "in vitro liquid assay" of at least 5 %, preferably of at least 10%, 20%, 30%, 40%, 50%, 60%, or 70%, 80%, more preferably of at least 90% and even more preferably of at least 95% and most preferably of at least 99% in comparison to a control to which no microorganism has been added.

Most preferably, a microorganism is regarded as inhibiting the growth of one or more microorganisms of the transient pathogenic skin micro flora if it leads to an decrease of growth of at least one such microorganism in an in situ skin assay of at least 5 %, preferably of at least 10%, 20%, 30%, 40%, 50%, 60%, or 70%, 80%, more preferably of at least 90%, even more preferably of at least 95 % and most preferably of at least 99 %.

The test for determining whether a microorganism inhibits or does not inhibit the growth of a microorganism of the transient pathogenic skin micro flora, e.g. *Staphylococcus aureus,* is preferably an in vitro and/or an in situ test as described herein-above, more preferably a test as described in the Examples.

In a preferred embodiment the microorganism according to the invention leads to an inhibition of the growth of one or more microorganisms of the pathogenic transient micro flora, preferably Staphylococcus aureus, which is comparable to the inhibition of growth of at least one such microorganism after the use of an antibiotic. The term "comparable" means that the inhibitory activity of a specific amount of the microorganism according to the invention is within the same range as the activity of an antibiotic. In particular, this effect can be achieved by using preferably an amount of between 1.0 x 10⁸ and 3.0 x 10⁹ cells, more preferably between 2.0 x 10⁸ and 1.0 x 10⁹ cells, even more preferably between 3.0 x 10⁸ and 5.0 x 10⁸ cells and most preferably at 3.4 x 10⁸ cells and the inhibitory activity achieved by this amount of cells corresponds preferably to 5 to 15 units of an antibiotic. The term "antibiotic" refers to a chemical substance which has the capacity to inhibit the growth or to kill microorganisms. Such substances are known to the person skilled in the art. Preferably, the term refers to beta-lactam compounds like penicillines, cephalosporins or carbapenems; macrolides; tetracyclines; fluoroquinolones; sulphonamides; aminoglycosides; imidazoles; peptide-antibiotics and lincosamides. More preferably, the term relates to bacitracin and erythromycin. In a preferred embodiment the term "comparable" means that the inhibitory activity of about 3.4 x 10⁸ cells of a microorganism of the present invention corresponds to about 150 µg of bacitracin or about 2.5 µg of erythromycin. Most preferably the term "comparable" reltates to the inhibitory activity of about 3.4 x 10⁸ cells of a microorganism of the present invention corresponds to about 150 µg of bacitracin or about 2.5 µg of erythromycin on *Staphylococcus aureus* as indicator strain, as illustrated in Example 12.

The term "microorganisms of the pathogenic transient micro flora" has been described herein above. Preferably, the term relates to *Staphylococcus aureus.*

The degree of growth inhibition of the microorganism(s) of the transient pathogenic skin micro flora in comparison to the inhibition of growth of at least one such microorganism after the use of an antibiotic can preferably be observed in vitro, more preferably in an assay in which a microorganism according to the invention is contacted with one or more microorganisms of the transient pathogenic skin micro flora and the growth of the(se) microorganism(s) of the transient pathogenic skin micro flora is determined. Most preferably, the comparison of growth inhibition can be determined in an "in vitro hole plate assay" as described in the Examples and mentioned herein above. In brief, such a comparison in an "in vitro hole plate assay" comprises the following steps
- cultivation of at least one microorganism of the transient pathogenic skin micro flora and evenly spreading it/them on a prepared agar plate containing a suitable agar medium for growth, and preferably detection, of the respective microorganism(s);
- providing holes in the inoculated agar plate;
- filling some of the holes with precultured cells of a microorganism according to the invention and filling some of the holes with an antibiotic at different concentrations;
- incubating the agar plates for an appropriate amount of time and under conditions allowing growth of the microorganism(s) of the transient pathogenic skin micro flora;
- determining the growth of the microorganism(s) of the transient pathogenic skin micro flora surrounding the holes containing a microorganism according to the invention and comparing it to the growth of the microorganism(s) surrounding a hole which contains an antibiotic at different concentrations;
- measurement of the diameter of the inhibition zones of the holes and calculation of the area of inhibition; and
- correlation of the growth inhibition caused by a microorganism according to the invention and an antibiotic.

In a preferred embodiment the term "inhibits the growth of microorganisms of the transient pathogenic skin micro flora" means that the decrease of growth of microorganisms of the transient pathogenic skin micro flora is due to the release of (defensive) antimicrobial substances. The term "antimicrobial substance" refers to a substance that is able to mediate the selective inhibition of growth of microorganisms of the transient pathogenic skin micro flora. Preferably the substance is not sensitive against protease digestion. The term "not sensitive" means that the substance is not or only partially affected by protease activity. The term "protease" refers to any enzyme that catalyses the splitting of interior peptide bonds in a protein, known to the person skilled in the art. In a preferred embodiment the term refers to proteinase K, a protease from *Streptomyces griseus,* trypsin or chymotrypsin. The term "protease digestion" refers to a protease reaction under conditions known to the person skilled in the art. In a preferred embodiment the term refers to an incubation at 37°C, for example for one our.

In a further preferred embodiment the term "antimicrobial substance" refers to a substance that is characterized by its property not to be disturbed at high or low pH values. The term "not to be disturbed" means that the substance is stable and biologically active. The terms "high pH value" and "low pH value" are known to the person skilled in the art. Preferably, the property not to be disturbed is present between pH 3 and pH 11.

The microorganism according to the invention is also characterized in that it does not inhibit the growth of the healthy normal resident skin micro flora. Thus, the terms "resident skin micro flora" and "healthy normal resident skin micro flora" relate to microorganisms which can normally be found on healthy skin, preferably human skin, and which constitute the majority of the microorganisms found on the skin.

In particular, the term "resident skin micro flora" relates to microorganisms which are permanent inhabitants on the surface of the skin, the stratum corneum and within the outer layer of the epidermis and the deeper crevices of the skin and hair follicles. These microorganisms are characterized in that they can grow and multiply on the skin without invading or damaging the skin tissue. A characteristic of these microorganisms is that washing does not easily remove them in deeper skin regions. The microorganisms of the resident skin micro flora are harmless commensals.

The term "resident skin micro flora" preferably relates to a flora of aerobic and anaerobic microorganisms which can be found on skin, preferably human skin. More preferably, it relates to a flora of microorganisms which comprises *Staphylococcus epidermidis* (coagulase negative), *Micrococcus* spec., Diphteroids and propioni bacteria. Typically, about 90 % of the aerobic resident microbial skin flora consists of *Staphylococcus epidermidis.* The remaining about 10 % are composed of mainly *Micrococcus spec.* (80 % *Micrococcus luteus*) and Diphteroids (13 %). The term "Diphtheroid" denotes a wide range of bacteria belonging to the genus *Corynebacterium*. For convenience, cutaneous diphtheroids have been categorized into the following four groups: lipophilic or nonlipophilic diphtheroids; anaerobic diphtheroids; diphtheroids producing porphyrins. Major representatives (90%) of the anaerobic microbial skin flora are propionibacteria; especially *Propionibacterium acnes, P. granulosum* and *P. avidum* can be isolated from the skin. The anaerobic flora accounts for approximately 4 % of the total resident skin flora.

More preferably, more than 90% of the microorganisms of the micro flora belong to *Staphylococcus epidermidis, Micrococcus* spec., Diphteroids and propioni bacteria. Even more preferably, the resident skin micro flora is characterized in that its major constituent is *Staphylococcus epidermidis.*

The term "skin" refers to the body's outer covering, as known to the person skilled in the art. Preferably the term relates to three layers: epidermis, dermis, and subcutaneous fatty tissue. The epidermis is the outermost layer of the skin. It typically forms the waterproof, protective wrap over the body's surface and is made up of stratified squamous epithelium with an underlying basal lamina. It usually contains no blood vessels, and is nourished by diffusion from the dermis. The main type of cells which make up the epidermis are keratinocytes, with melanocytes and Langerhans cells also present. The epidermis is divided into several layers where cells are formed through mitosis at the innermost layers. They move up the strata changing shape and composition as they differentiate and become filled with keratin. They eventually reach the top layer called stratum corneum and become sloughed off, or desquamated. The outermost layer of the epidermis consists of 25 to 30 layers of dead cells. Conventionally, the epidermis is divided into 5 sublayers or strata (from superficial to deep): the stratum corneum, the stratum lucidum, the stratum granulosum, the stratum spinosum and the stratum germinativum or stratum basale. Typically, the interface between the epidermis and dermis is irregular and consists of a succession of papillae, or fingerlike projections, which are smallest where the skin is thin and longest in the skin of the palms and soles. Typically, the papillae of the palms and soles are associated with elevations of the epidermis, which produce ridges. Subcutaneous fatty tissue is the deepest layer of the skin. A characteristic of this layer is that it is composed of connective tissue, blood vessels, and fat cells. Typically, this layer binds the skin to underlying structures, insulates the body from cold, and stores energy in the form of fat. In general the skin forms a protective barrier against the action of physical, chemical, and bacterial agents on the deeper tissues. This means that tissues belonging , e.g. to the oral cavity or the vaginal region or mucous membranes do not belong to the skin. In a preferred embodiment the term "skin" relates to the outermost layer of the body's covering, i.e. the epidermis. In a more preferred embodiment the term "skin" relates to the stratum corneum of the epidermis. In an even more preferred embodiment the term skin relates to the outermost 25 to 30 layers of dead cells of the epidermis. In the most preferred embodiment the term "skin" relates to the outermost 10 layers of dead cell of the epidermis

The term "not inhibit" in connection with the growth of microorganisms of the resident skin micro flora means that the growth of at least one, preferably of more than one, preferably of more than two, more preferably of more than five and particularly preferred of any of the microorganisms of the resident skin micro flora is not altered when contacted with a microorganism according to the invention. A not altered growth means preferably an unchanged proliferation, i.e. cell divisions per time unit. A microorganism is regarded as not altering the growth of a microorganism of the resident skin micro flora if it does not lead to an decreased growth of such a microorganism of the resident skin micro flora when contacted with it. The inhibition of growth or its absence can be tested in vitro or in situ as described above in connection with the property of a microorganism of the invention to inhibit the growth of at least one microorganism of the transient pathogenic skin micro flora. Most preferably the test for determining inhibition or its absence takes place by carrying out an "in vitro hole plate assay" and/or "in vitro liquid assay" and/or an "in situ skin assay" with a microorganism of the resident skin micro flora as explained herein below, more preferably as described in the Examples.

In brief, an "in vitro hole plate assay" with a microorganism of the resident skin micro flora comprises the following steps:
- cultivation of at least one microorganism of the resident skin microbial flora and evenly spreading it/them on a prepared agar plate containing a suitable agar medium for growth, and preferably detection, of the respective microorganism(s);
- providing holes in the inoculated agar plate;
- filling the holes with precultured cells of a microorganism according to the invention;
- incubating the agar plates for an appropriate amount of time and under conditions allowing growth of the microorganism(s) of the resident skin microbial flora; and
- determining the growth of the microorganism(s) of the resident skin microbial flora surrounding the holes containing a microorganism according to the invention and comparing it to the growth of the microorganism(s) surrounding a hole which does not contain a microorganism according to the invention.

The determination of the growth in the last step may be effected by available means and methods for determining the number of cells and/or colonies, e.g. by staining with an appropriate dye and/or optical means such as densitometry and counting the cells/colonies under the microscope. In a preferred embodiment the diameter of the occurring clearing zone next to the hole may be used to determine the area of inhibition.

An assay "in vitro liquid assay" with a microorganism of the resident skin micro flora is described in the Examples and, briefly, comprises the following steps:
- cultivation of at least one microorganism of the resident skin micro flora in a liquid culture;
- applying an aliquot of a liquid culture of the microorganism according to the invention and an aliquot of a liquid culture of the microorganism of the resident skin micro flora to a culture medium allowing the growth of the microorganism of the resident skin micro flora;
- co-cultivation of the microorganism according to the invention and the microorganism of the resident skin micro flora in a liquid culture;
- transferring an aliquot of the co-cultivation liquid culture to an agar plate, containing an appropriate growth medium;
- incubation of the agar plates for a period of time and under conditions allowing the growth of the microorganism(s) of the resident skin micro flora;
- determining the growth of the microorganism(s) of the resident skin micro flora by quantification of the colony forming units and comparing it to the growth of the microorganism(s) in a control in which no microorganism of the invention was applied.

In brief, an "in situ skin assay" with a microorganism of the resident skin micro flora comprises the following steps:
- cultivation of at least one microorganism of the resident skin micro flora and evenly spreading it on an area of skin of a test individual;
- applying an aliquot of a microorganism according to the invention in a punctual area within the area on which the microorganism(s) of the resident skin micro flora has/have been spread;
- incubating the skin for an amount of time sufficient to allow growth of the microorganism(s) of the resident skin micro flora;
- transferring the upper skin layers, including the microorganisms comprised in these, to an agar plate containing an appropriate growth medium;
- incubation of the agar plates for a period of time and under conditions allowing the growth of the microorganism(s) of the resident skin micro flora;
- determining the growth of the microorganism(s) of the resident skin micro flora surrounding the area at which the microorganism according to the invention was applied and comparing it to the growth of the microorganism(s) in a control in which no microorganism of the invention was applied.

A microorganism is regarded as not altering the growth of a microorganism of the resident skin micro flora if the growth of the latter microorganism is not decreased or only slightly decreased when contacted with the former microorganism. "Slightly decreased" means that the growth is decreased not more than by 5% when compared to the control, more preferably not more than 2% when compared to the control. The term "not decreased" means that there can be found no statistically relevant difference between the growth of the microorganism of the resident skin micro flora contacted with a microorganism of the invention when compared to the control where no microorganism of the invention is present. The term "not decreased" in a preferred embodiment also includes those cases where a microorganism actually leads to an increase of the growth of a microorganism of the resident skin micro flora, i.e. where it stimulates the growth of such a microorganism.

In another preferred embodiment the microorganism of the present invention does not negatively influence the growth of the microorganisms of the resident skin micro flora. The term "not negatively influence" means that that there can be found no inhibition of the growth of the microorganism of the resident skin micro flora contacted with a microorganism of the invention when compared to the control where no microorganism of the invention is present.

The microorganism of the present invention is a microorganism belonging to the group of lactic acid bacteria. The term "microorganism belonging to the group of lactic acid bacteria" encompasses (a) microorganism(s) which belong(s) to bacteria, in particular belonging to gram-positive fermentative eubacteria, more particularly belonging to the family of lactobacteriaceae including lactic acid bacteria. Lactic acid bacteria are from a taxonomical point of view divided up into the subdivisions of Streptococcus, Leuconostoc, Pediococcus, Lactococcus and Lactobacillus. The microorganism of the present invention is a Lactobacillus species. Members of the lactic acid bacteria group normally lack porphyrins and cytochromes, do not carry out electron-transport phosphorylation and hence obtain energy only by substrate-level phosphorylation. I.e. in lactic acid bacteria ATP is synthesized through fermentation of carbohydrates. All of the lactic acid bacteria grow anaerobically, however, unlike many anaerobes, most lactic acid bacteria are not sensitive to oxygen and can thus grow in its presence as well as in its absence. Accordingly, the bacteria of the present invention are aerotolerant anaerobic lactic acid bacteria, preferably belonging to the genus of Lactobacillus.

The microorganisms of the present invention can be classified to belong to the group of lactic acid bacteria, particularly to the genus of Lactobacillus. By using classical systematics, for example, by reference to the pertinent descriptions in "Bergey's Manual of Systematic Bacteriology" (Williams & Wilkins Co., 1984), a microorganism of the present invention can be determined to belong to the genus of Lactobacillus. Alternatively, the microorganisms of the present invention can be classified to belong to the genus of Lactobacillus by methods known in the art, for example, by their metabolic fingerprint, i.e. a comparable overview of the capability of the microorganism(s) of the present invention to metabolize sugars or by other methods described, for example, in Schleifer et al., System. Appl. Microb., 18 (1995), 461-467 or Ludwig et al., System. Appl. Microb., 15 (1992), 487-501. The microorganisms of the present invention are capable of metabolizing sugar sources which are typical and known in the art for microorganisms belonging to the genus of Lactobacillus.

The affiliation of the microorganisms of the present invention to the genus of Lactobacillus can also be characterized by using other methods known in the art, for example, using SDS-PAGE gel electrophoresis of total protein of the species to be determined and comparing them to known and already characterized strains of the genus Lactobacillus. The techniques for preparing a total protein profile as described above, as well as the numerical analysis of such profiles, are well known to a person skilled in the art. However, the results are only reliable insofar as each stage of the process is sufficiently standardized. Faced with the requirement of accuracy when determining the attachment of a microorganism to the genus of Lactobacillus, standardized procedures are regularly made available to the public by their authors such as that of Pot et al., as presented during a "workshop" organized by the European Union, at the University of Ghent, in Belgium, on Sep. 12 to 16, 1994 (Fingerprinting techniques for classification and identification of bacteria, SDS-PAGE of whole cell protein). The software used in the technique for analyzing the SDS-PAGE electrophoresis gel is of crucial importance since the degree of correlation between the species depends on the parameters and algorithms used by this software. Without going into the theoretical details, quantitative comparison of bands measured by a densitometer and normalized by a computer is preferably made with the Pearson correlation coefficient. The similarity matrix thus obtained may be organized with the aid of the UPGMA (unweighted pair group method using average linkage) algorithm that not only makes it possible to group together the most similar profiles, but also to construct dendograms (see Kersters, Numerical methods in the classification and identification of bacteria by electrophoresis, in Computer-assisted Bacterial Systematics, 337-368, M. Goodfellow. A. G. O'Donnell Ed., John Wiley and Sons Ltd, 1985).

Alternatively, the affiliation of said microorganisms of the present invention to the genus of Lactobacillus can be characterized with regard to ribosomal RNA in a so called Riboprinter.RTM. More preferably, the affiliation of the newly identified species of the invention to the genus Lactobacillus is demonstrated by comparing the nucleotide sequence of the 16S ribosomal RNA of the bacteria of the invention, or of their genomic DNA which codes for the 16S ribosomal RNA, with those of other genera and species of lactic acid bacteria known to date. Another preferred alternative for determining the attachment of the newly identified species of the invention to the genus Lactobacillus is the use of species-specific PCR primers that target the 16S-23S rRNA spacer region. Another preferred alternative is RAPD-PCR (Nigatu et al. in Antonie van Leeuwenhoek (79), 1-6, 2001) by virtue of that a strain specific DNA pattern is generated which allows to determine the affiliation of an identified microorganisms in accordance with the present invention to the genus of Lactobacillus. Further techniques useful for determining the affiliation of the microorganism of the present invention to the genus of Lactobacillus are restriction fragment length polymorphism (RFLP) (Giraffa et al., Int. J. Food Microbiol. 82 (2003), 163-172), fingerprinting of the repetitive elements (Gevers et al., FEMS Microbiol. Lett. 205 (2001) 31-36) or analysis of the fatty acid methyl ester (FAME) pattern of bacterial cells (Heyrman et al., FEMS Microbiol. Lett. 181 (1991), 55-62). Alternatively, lactobacilli can be determined by lectin typing (Annuk et al., J. Med. Microbiol. 50 (2001), 1069-1074) or by analysis of their cell wall proteins (Gatti et al., Lett. Appl. Microbiol. 25 (1997), 345-348.

In a preferred embodiment of the present application the microorganism is a probiotic Lactobacillus species. The term "probiotic" in the context of the present invention means that the microorganism has a beneficial effect on health if it is topically applied to the skin. Preferably, a "probiotic" microorganism is a live microorganism which, when topically applied to the skin, is beneficial for health of this tissue. Most preferably, this means that the microorganism has a positive effect on the micro flora of the skin.

The microorganism of the present invention belongs to the species of *Lactobacillus buchneri* or *Lactobacillus delbrückii.*

In particular, the microorganism of the present invention is selected from the group consisting of *Lactobacillus buchneri,* or *Lactobacillus delbrückii* being deposited at the DSMZ under the accession number DSM **18007** (*Lactobacillus buchneri* OB-LB-Sa16) and DSM **18006** (*Lactobacillus delbrückii ssp. delbrückii* OB-LB-Sa3). The invention also relates to a mutant of the above-mentioned deposited Lactobacillus strains wherein said mutants or derivatives have retained their capability according to claim 1.

The term *"Lactobacillus buchneri* or *Lactobacillus delbrückii* being deposited at the DSMZ under the accession number" relates to cells of a microorganism belonging to the species *Lactobacillus buchneri,* or *Lactobacillus delbrückii* deposited at the Deutsche Sammlung für Mikroorganismen und Zellkulturen (DSMZ) on February 24, 2006 and having the following deposit numbers: DSM **18007** (*Lactobacillus buchneri* OB-LB-Sa16) and DSM **18006** (*Lactobacillus delbrückii ssp. delbrückii* OB-LB-Sa3). The DSMZ is located at the Mascheroder Weg 1b, D-38124 Braunschweig, Germany. The aforementioned deposits were made pursuant to the terms of the Budapest treaty on the international recognition of the deposit of microorganisms for the purposes of patent procedures.

In a particular preferred embodiment the microorganisms of the present invention are "isolated" or "purified". The term "isolated" means that the material is removed from its original environment, e.g. the natural environment if it is naturally occurring, or the culture medium if it is cultured. For example, a naturally-occurring microorganism separated from some or all of the coexisting materials in the natural system, is isolated. Such a microorganism could be part of a composition, and is to be regarded as still being isolated in that the composition is not part of its natural environment. The term "purified" does not require absolute purity; rather, it is intended as a relative definition. Individual microorganisms obtained from a library have been conventionally purified to microbiological homogeneity, i.e. they grow as single colonies when streaked out on agar plates by methods known in the art. Preferably, the agar plates that are used for this purpose are selective for Lactobacillus species. Such selective agar plates are known in the art.

In another aspect the present invention relates to an inactivated form of the microorganism of the present invention, which is thermally inactivated or lyophilized, but which retains the property of inhibiting the growth of one or more microorganisms of the transient pathogenic skin micro flora and of not inhibiting the growth of microorganisms of the healthy normal resident skin micro flora.

According to the present invention the term "inactivated form of the microorganism of the present invention" includes a dead or inactivated cell of the microorganism of the present invention, which is no longer capable to form a single colony on a plate specific for microorganisms belonging to the genus of Lactobacillus. Said dead or inactivated cell may have either an intact or broken cell membrane. Methods for killing or inactivating cells of the microorganism of the present invention are known in the art. El-Nezami et al., J. Food Prot. 61 (1998), 466-468 describes a method for inactivating Lactobacillus species by UV-irradiation. Specifically, the cells of the microorganism of the present invention are thermally inactivated or lyophilised. Lyophilisation of the cells of the present invention has the advantage that they can be easily stored and handled while retaining their property of inhibiting the growth of one or more microorganisms of the transient pathogenic skin micro flora and of not inhibiting the growth of microorganisms of the healthy normal resident skin micro flora.. Moreover, lyophilised cells can be grown again when applied under conditions known in the art to appropriate liquid or solid media. Lyophilization is done by methods known in the art. Preferably, it is carried out for at least 2 hours at room temperature, i.e. any temperature between 16°C and 25°C. Moreover, the lyophilized cells of the microorganism of the present invention are stable for at least 4 weeks at a temperature of 4°C so as to still retain their properties as described above. Thermal inactivation can be achieved by incubating the cells of the microorganism of the present invention for at least 2 hours at a temperature of 170°C. Yet, thermal inactivation is preferably achieved by autoclaving said cells at a temperature of 121 °C for at least 20 minutes in the presence of satured steam at an atmospheric pressure of 2 bar. In the alternative, thermal inactivation of the cells of the microorganism of the present invention is achieved by freezing said cells for at least 4 weeks, 3 weeks, 2 weeks, 1 week, 12 hours, 6 hours, 2 hours or 1 hour at -20°C. It is preferred that at least 70%, 75% or 80%, more preferably 85%, 90% or 95% and particularly preferred at least 97%, 98%, 99% and more particularly preferred, 99.1%, 99.2%, 99.3%, 99.4%, 99.5%, 99.6%, 99.7%, 99.8% or 99.9% and most particularly preferred 100% of the cells of the inactivated form of the microorganism of the present invention are dead or inactivated, however, they have still the capability to inhibit the growth of one or more microorganisms of the transient pathogenic skin micro flora but do not inhibit the growth of microorganisms of the healthy normal resident skin micro flora. Whether the inactivated form of the microorganism of the present invention is indeed dead or inactivated can be tested by methods known in the art, for example, by a test for viability.

In another aspect the present invention relates to a composition comprising a microorganism according to the present invention or a mutant or inactive form of this microorganism as described above. In a preferred embodiment, said composition comprises a microorganism as described above in an amount between 10² to 10¹² cells, preferably 10³ to 10⁸ cells per mg in a solid form of the composition. In case of a liquid form of compositions, the amount of the microorganisms is between 10² to 10¹³ cells per ml. In a further preferred embodiment said compositions are in the form of emulsions, e.g. oil in water or water in oil emulsions, in the form of ointments or in the form of micro- capsules. In case of emulsions, ointments or microcapsules the compositions comprise a microorganism as described herein in an amount between 10² to 10¹³ cells per ml. However, for specific compositions the amount of the microorganism may be different as is described herein.

In a still further aspect, the present invention provides a method for the production of a composition for protecting the skin against pathogenic microorganisms comprising the steps of formulating a microorganism according to the invention or a mutant or inactive form of this microorganism as described above with a cosmetically or pharmaceutical acceptable carrier or excipient.

The term "composition", as used in accordance with the present invention, relates to (a) composition(s) which comprise(s) at least one microorganism of the present invention or mutant, derivative or inactive form of said microorganism as described above. It is envisaged that the compositions of the present invention which are described herein below comprise the aforementioned ingredients in any combination. It may, optionally, comprise at least one further ingredient suitable for protecting the skin against pathogenic microorganisms. Accordingly, it may optionally comprise any combination of the hereinafter described further ingredients. The term "ingredients suitable for protecting the skin against pathogenic microorganisms" encompasses compounds or compositions and/or combinations thereof which lower the pH.

The composition may be in solid, liquid or gaseous form and may be, inter alia, in the form of (a) powder(s), (a) solution(s) (an) aerosol(s), suspensions, emulsions, liquids, elixirs, extracts, tincture or fluid extracts or in a form which is particularly suitable for topical administration. Forms suitable for topical application include, e.g., a paste, an ointment, a lotion, a cream, a gel or a transdermal patch.

Preferably, the composition of the present invention is a cosmetic composition further comprising a cosmetically acceptable carrier or excipient. More preferably, said cosmetic composition is a paste, an ointment, a lotion, a cream or a gel.

The cosmetic composition of the present invention comprises the microorganism of the present invention, mutant or inactive form thereof as described above in connection with the composition of the invention and further a cosmetically acceptable carrier. Preferably the cosmetic composition of the present invention is for use in topical applications.

The term "cosmetically acceptable carrier" as used herein means a suitable vehicle, which can be used to apply the present compositions to the skin in a safe and effective manner. Such vehicle may include materials such as emulsions, e.g. oil in water or water in oil emulsions, ointments or micro capsules. It is also advantageous to administer the active ingredients in encapsulated form, e.g. as cellulose encapsulation, in gelatine, with polyamides, niosomes, wax matrices, with cyclodextrins or liposomally encapsulated. The term "safe and effective amount" as used herein, means a sufficient amount to inhibit the growth of one or more microorganisms of the transient pathogenic skin micro flora.

In another aspect the present invention relates to a pharmaceutical composition comprising the microorganism of the present invention or a mutant or an inactive form thereof as described above further comprising a pharmaceutical acceptable carrier or excipient. The pharmaceutical composition preferably is in a form which is suitable for topical administration.

In addition, the present invention relates to the use of a microorganism of the present invention or of a mutant or an inactive form thereof as described above for the preparation of a composition, preferably a pharmaceutical or cosmetic composition.

Pharmaceutical compositions comprise a therapeutically effective amount of a microorganism of the present invention or of a derivative or mutant of the present invention or an inactive form of said microorganism of the present invention as described above and can be formulated in various forms, e.g. in solid, liquid, powder, aqueous, lyophilized form.

The pharmaceutical composition may be administered with a pharmaceutically acceptable carrier to a patient, as described herein. In a specific embodiment, the term "pharmaceutically acceptable" means approved by a regulatory agency or other generally recognized pharmacopoeia for use in animals, and more particularly in humans.

The term "carrier" refers to a diluent, adjuvant, excipient, or vehicle with which the therapeutic is administered. Such a carrier is pharmaceutically acceptable, i.e. is non-toxic to a recipient at the dosage and concentration employed. It is preferably isotonic, hypotonic or weakly hypertonic and has a relatively low ionic strength, such as provided by a sucrose solution. Such pharmaceutical carriers can be sterile liquids, such as water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers. Suitable pharmaceutical excipients include starch, glucose, sucrose, gelatin, malt, rice, flour, chalk, silica gel, sodium stearate, glycerol monostearate, talc, sodium ion, dried skim milk, glycerol, propylene, glycol, water, ethanol and the like. The composition, if desired, can also contain minor amounts of wetting or emulsifying agents, or pH buffering agents. These compositions can take the form of, e.g., solutions, suspensions, emulsion, powders, sustained-release formulations and the like. Examples of suitable pharmaceutical carriers are described in "Remington's Pharmaceutical Sciences" by E.W. Martin. Some other examples of substances which can serve as pharmaceutical carriers are sugars, such as glucose and sucrose; starches such as corn starch and potato starch; cellulose and its derivatives such as sodium carboxymethycellulose, ethylcellulose and cellulose acetates; powdered tragancanth; malt; gelatin; talc; stearic acids; magnesium stearate; calcium sulfate; calcium carbonate; vegetable oils, such as peanut oils, cotton seed oil, sesame oil, olive oil, corn oil and oil of theobroma; polyols such as propylene glycol, glycerine, sorbitol, manitol, and polyethylene glycol; agar; alginic acids; pyrogen-free water; isotonic saline; cranberry extracts and phosphate buffer solution; skim milk powder; as well as other non-toxic compatible substances used in pharmaceutical formulations such as Vitamin C, estrogen and echinacea, for example. Wetting agents and lubricants such as sodium lauryl sulfate, as well as coloring agents, flavoring agents, lubricants, excipients, tabletting agents, stabilizers, anti-oxidants and preservatives, can also be present. It is also advantageous to administer the active ingredients in encapsulated form, e.g. as cellulose encapsulation, in gelatine, with polyamides, niosomes, wax matrices, with cyclodextrins or liposomally encapsulated.

Generally, the ingredients are supplied either separately or mixed together in unit dosage form, for example, as a dry lyophilised powder or water free concentrate in a hermetically sealed container such as an ampoule or sachette indicating the quantity of active agent.

The pharmaceutical composition of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

In vitro or in situ assays, e.g. those described in the Examples, may optionally be employed to help identify optimal dosage ranges. The precise dose to be employed in the formulation will also depend on the route of administration, and the seriousness of the disease or disorder, and should be decided according to the judgment of the practitioner and each patient's circumstances. The topical route of administration is preferred. Effective doses may be extrapolated from dose-response curves derived from in vitro or (animal) model test systems. Preferably, the pharmaceutical composition is administered directly or in combination with an adjuvant. Adjuvants may be selected from the group consisting of a chloroquine, protic polar compounds, such as propylene glycol, polyethylene glycol, glycerol, EtOH, 1-methyl L-2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or their derivatives. These compounds are added in conditions respecting pH limitations. The composition of the present invention can be administered to a vertebrate. "Vertebrate" as used herein is intended to have the same meaning as commonly understood by one of ordinary skill in the art. Particularly, "vertebrate" encompasses mammals, and more particularly humans.

The term "administered" means administration of a therapeutically effective dose of the aforementioned composition. By "therapeutically effective amount" is meant a dose that produces the effects for which it is administered, preferably this effect is the protection of skin against pathogenic microorganisms. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

The methods are applicable to both human therapy and veterinary applications. The compounds described herein having the desired therapeutic activity may be administered in a physiologically acceptable carrier to a patient, as described herein. Depending upon the manner of administration, the compounds may be formulated in a variety of ways as discussed below. The concentration of the therapeutically active compound in the formulation may vary from about 0.01-100 wt %. The agent may be administered alone or in combination with other treatments.

The administration of the pharmaceutical composition can be done in a variety of ways. The preferable route of administering is the topical route.

The attending physician and clinical factors will determine the dosage regimen. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg; however, doses below or above this exemplary range are envisioned, especially considering the aforementioned factors.

The dosages are preferably given once a week, more preferably 2 times, 3 times, 4 times, 5 times or 6 times a week and most preferably daily and even more preferably, 2 times a day or more often. In particular, it may be preferable to give a dosage each time after a disturbance of the resident skin flora occurred, e.g. by washing. However, during progression of the treatment the dosages can be given in much longer time intervals and in need can be given in much shorter time intervals, e.g., several times a day. In a preferred case the immune response is monitored using herein described methods and further methods known to those skilled in the art and dosages are optimized, e.g., in time, amount and/or composition. Progress can be monitored by periodic assessment. It is also envisaged that the pharmaceutical compositions are employed in co-therapy approaches, i.e. in co-administration with other medicaments or drugs, for example other drugs for protecting skin against pathogenic microorganisms.

Topical administration of the cosmetic or pharmaceutical composition of the present invention is useful when the desired treatment involves areas or organs readily accessible by topical administration. For application topically to the skin, the pharmaceutical composition is preferably formulated with a suitable paste, ointment, lotion, cream, gel or transdermal patches. The cosmetic or pharmaceutical preparations can, depending on the field of use, also be in the form of a spray (pump spray or aerosol), foam, gel spray, mousse, suspensions or powders.

A suitable paste comprises the active ingredient suspended in a carrier. Such carriers include, but are not limited to, petroleum, soft white paraffin, yellow petroleum jelly and glycerol.

The cosmetic or pharmaceutical composition may also be formulated with a suitable ointment comprising the active components suspended or dissolved in a carrier. Such carriers include, but are not limited to, one or more of glycerol, mineral oil, liquid oil, liquid petroleum, white petroleum, yellow petroleum jelly, propylene glycol, alcohols, triglycerides, fatty acid esters such as cetyl ester, polyoxyethylene polyoxypropylene compound, waxes such as white wax and yellow beeswax, fatty acid alcohols such as cetyl alcohol, stearyl alcohol and cetylstearylalcohol, fatty acids such as stearic acid, cetyl stearate, lanolin, magnesium hydroxide, kaolin and water. Alternatively, the cosmetic or pharmaceutical composition may also be formulated with a suitable lotion or cream comprising the active components suspended or dissolved in a carrier. Such carriers include, but are not limited to, one or more of mineral oil such as paraffin, vegetable oils such as castor oil, castor seed oil and hydrogenated castor oil, sorbitan monostearat, polysorbat, fatty acid esters such as cetyl ester, wax, fatty acid alcohols such as cetyl alcohol, stearyl alcohol, 2-octyldodecanol, benzyl alcohol, alcohols, triglycerides and water.

Alternatively, the cosmetic or pharmaceutical composition may also be formulated with a suitable gel comprising the active components suspended or dissolved in a carrier. Such carriers include, but are not limited to, one or more of water, glycerol, propyleneglycole, liquid paraffin, polyethylene, fatty oils, cellulose derivatives, bentonite and colloidal silicon dioxide.

Suitable propellants for aerosols according to the invention are the customary propellants, for example propane, butane, pentane and others.

The preparations according to the invention may generally comprise further auxiliaries as are customarily used in such preparations, e.g. preservatives, perfumes, antifoams, dyes, pigments, thickeners, surface-active substances, emulsifiers, emollients, finishing agents, fats, oils, waxes or other customary constituents, of a cosmetic or dermatological formulation, such as alcohols, polyols, polymers, foam stabilizers, solubility promoters, electrolytes, organic acids, organic solvents, or silicone derivatives.

The cosmetic or pharmaceutical composition according to the invention may comprise emollients. Emollients may be used in amounts which are effective to prevent or relieve dryness. Useful emollients include, without limitation: hydrocarbon oils and waxes; silicone oils; triglyceride esters; acetoglyceride esters; ethoxylated glyceride; alkyl esters; alkenyl esters; fatty acids; fatty alcohols; fatty alcohol ethers; etheresters; lanolin and derivatives; polyhydric alcohols (polyols) and polyether derivatives; polyhydric alcohol (polyol) esters; wax esters; beeswax derivatives; vegetable waxes; phospholipids; sterols; and amides.

Thus, for example, typical emollients include mineral oil, especially mineral oils having a viscosity in the range of 50 to 500 SUS, lanolin oil, mink oil, coconut oil, cocoa butter, olive oil, almond oil, macadamia nut oil, aloa extract, jojoba oil, safflower oil, corn oil, liquid lanolin, cottonseed oil, peanut oil, purcellin oil, perhydrosqualene (squalene), caster oil, polybutene, odorless mineral spirits, sweet almond oil, avocado oil, calophyllum oil, ricin oil, vitamin E acetate, olive oil, mineral spirits, cetearyl alcohol (mixture of fatty alcohols consisting predominantly of cetyl and stearyl alcohols), linolenic alcohol, oleyl alcohol, octyl dodecanol, the oil of cereal germs such as the oil of wheat germ cetearyl octanoate (ester of cetearyl alcohol and 2-ethylhexanoic acid), cetyl palmitate, diisopropyl adipate, isopropyl palmitate, octyl palmitate, isopropyl myristate, butyl myristate, glyceryl stearate, hexadecyl stearate, isocetyl stearate, octyl stearate, octylhydroxy stearate, propylene glycol stearate, butyl stearate, decyl oleate, glyceryl oleate, acetyl glycerides, the octanoates and benzoates of (C12-C15) alcohols, the octanoates and decanoates of alcohols and polyalcohols such as those of glycol and glycerol, and ricin- oleates of alcohols and poly alcohols such as those of isopropyl adipate, hexyl laurate, octyl dodecanoate, dimethicone copolyol, dimethiconol, lanolin, lanolin alcohol, lanolin wax, hydrogenated lanolin, hydroxylated lanolin, acetylated lanolin, petrolatum, isopropyl lanolate, cetyl myristate, glyceryl myristate, myristyl myristate, myristyl lactate, cetyl alcohol, isostearyl alcohol stearyl alcohol, and isocetyl lanolate, and the like.

Moreover, the cosmetic or pharmaceutical composition according to the invention may also comprise emulsifiers. Emulsifiers (i.e., emulsifying agents) are preferably used in amounts effective to provide uniform blending of ingredients of the composition. Useful emulsifiers include (i) anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium cetyl sulfate; and polyol fatty acid monoesters containing sulfuric esters, e.g., glyceryl monostearate containing sodium lauryl surfate; (ii) cationics chloride such as N(stearoyl colamino formylmethyl) pyridium; N-soya-N-ethyl morpholinium ethosulfate; alkyl dimethyl benzyl ammonium chloride; diisobutylphenoxytheoxyethyl dimethyl benzyl ammonium chloride; and cetyl pyridium chloride; and (iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate; and polyol fatty acid esters, e.g., glyceryl monostearate and propylene glycol monostearate; and ethoxylated lanolin derivatives, e.g., ethoxylated lanolins, ethoxylated lanolin alcohols and ethoxylated cholesterol. The selection of emulsifiers is exemplarly described in Schrader, Grundlagen und Rezepturen der Kosmetika, Hüthig Buch Verlag, Heidelberg, 2nd edition, 1989, 3rd part.

The cosmetic or pharmaceutical composition according to the invention may also include a surfactant. Suitable surfactants may include, for example, those surfactants generally grouped as cleansing agents, emulsifying agents, foam boosters, hydrotropes, solubilizing agents, suspending agents and nonsurfactants (facilitates the dispersion of solids in liquids).

The surfactants are usually classified as amphoteric, anionic, cationic and nonionic surfactants. Amphoteric surfactants include acylamino acids and derivatives and N-alkylamino acids. Anionic surfactants include: acylamino acids and salts, such as, acylglutamates, acylpeptides, acylsarcosinates, and acyltaurates; carboxylic acids and salts, such as, alkanoic acids, ester carboxylic acids, and ether carboxylic acids; sulfonic acids and salts, such as, acyl isethionates, alkylaryl sulfonates, alkyl sulfonates, and sulfosuccinates; sulfuric acid esters, such as, alkyl ether sulfates and alkyl sulfates. Cationic surfactants include: alkylamines, alkyl imidazolines, ethoxylated amines, and quaternaries (such as, alkylbenzyldimethylammonium salts, alkyl betaines, heterocyclic ammonium salts, and tetra alkylammonium salts). And nonionic surfactants include: alcohols, such as primary alcohols containing 8 to 18 carbon atoms; alkanolamides such as alkanolamine derived amides and ethoxylated amides; amine oxides; esters such as ethoxylated carboxylic acids, ethoxylated glycerides, glycol esters and derivatives, monoglycerides, polyglyceryl esters, polyhydric alcohol esters and ethers, sorbitan/sorbitol esters, and triesters of phosphoric acid; and ethers such as ethoxylated alcohols, ethoxylated lanolin, ethoxylated polysiloxanes, and propoxylated polyoxyethylene ethers.

Furthermore, a cosmetic or pharmaceutical composition according to the invention may also comprise a film former. Suitable film formers which are used in accord with the invention keep the composition smooth and even and include, without limitation: acrylamide/sodium acrylate copolymer; ammonium acrylates copolymer; Balsam Peru; cellulose gum; ethylene/maleic anhydride copolymer; hydroxyethylcellulose; hydroxypropylcellulose; polyacrylamide; polyethylene; polyvinyl alcohol; pvm/MA copolymer (polyvinyl methylether/maleic anhydride); PVP (polyvinylpyrrolidone); maleic anhydride copolymer such as PA-18 available from Gulf Science and Technology; PVP/hexadecene copolymer such as Ganex V-216 available from GAF Corporation; acryliclacrylate copolymer; and the like.

Generally, film formers can be used in amounts of about 0.1% to about 10% by weight of the total composition with about 1% to about 8% being preferred and about 0.1 DEG/O to about 5% being most preferred. Humectants can also be used in effective amounts, including: fructose; glucose; glulamic acid; glycerin; honey; maltitol; methyl gluceth-10; methyl gluceth-20; propylene glycol; sodium lactate; sucrose; and the like.

Of course, the cosmetic or pharmaceutical composition of the present invention can also comprise a preservative. Preservatives according to certain compositions of the invention include, without limitation: butylparaben; ethylparaben; imidazolidinyl urea; methylparaben; O-phenylphenol; propylparaben; quaternium-14; quaternium-15; sodium dehydroacetate; zinc pyrithione; and the like.

The preservatives are used in amounts effective to prevent or retard microbial growth. Generally, the preservatives are used in amounts of about 0.1 % to about 1% by weight of the total composition with about 0.1% to about 0.8% being preferred and about 0.1 % to about 0.5% being most preferred.

A cosmetic or pharmaceutical composition according to the invention may also comprise a perfume. Perfumes (fragrance components) and colorants (coloring agents) well known to those skilled in the art may be used in effective amounts to impart the desired fragrance and color to the compositions of the invention.

Furthermore, a cosmetic or pharmaceutical composition of the present invention may also comprise a wax. Suitable waxes which are useful in accord with the invention include: animal waxes, such as beeswax, spermaceti, or wool wax (lanolin); plant waxes, such as carnauba or candelilla; mineral waxes, such as montan wax or ozokerite; and petroleum waxes, such as paraffin wax and microcrystalline wax (a high molecular weight petroleum wax). Animal, plant, and some mineral waxes are primarily esters of a high molecular weight fatty alcohol with a high molecular weight fatty acid. For example, the hexadecanoic acid ester of tricontanol is commonly reported to be a major component of beeswax. Other suitable waxes according to the invention include the synthetic waxes including polyethylene polyoxyethylene and hydrocarbon waxes derived from carbon monoxide and hydrogen.

Representative waxes also include: cerosin; cetyl esters; hydrogenated joioba oil; hydrogenated jojoba wax; hydrogenated rice bran wax; Japan wax; jojoba butter; jojoba oil; jojoba wax; munk wax; montan acid wax; ouricury wax; rice bran wax; shellac wax; sufurized jojoba oil; synthetic beeswax; synthetic jojoba oils; trihydroxystearin; cetyl alcohol; stearyl alcohol; cocoa butter; fatty acids of lanolin; mono-, di- and 25 triglycerides which are solid at 25 DEG C., e.g., glyceyl tribehenate (a triester of behenic acid and glycerine) and C1g-C36 acid triglyceride (a mixture of triesters of C1g-C36 carboxylic acids and glycerine) available from Croda, Inc., New York, N.Y. under the tradenames Syncrowax HRC and Syncrowax HGL-C, respectively; fatty esters which are solid at 25 DEG C.; silicone waxes such as methyloctadecaneoxypolysiloxane and poly (dimethylsiloxy) stearoxysiloxane; stearyl mono- and diethanolamide; rosin and its derivatives such as the abietates of glycol and glycerol; hydrogenated oils solid at 25 DEG C.; and sucroglycerides. Thickeners (viscosity control agents) which may be used in effective amounts in aqueous systems include: algin; carbomers such as carbomer 934, 934P, 940 and 941; cellulose gum; cetearyl alcohol, cocamide DEA, dextrin; gelatin; hydroxyethylcellulose; hydroxypropylcellulose; hydroxypropyl methylcellulose; magnesium aluminum silicate; myristyl alcohol; oat flour; oleamide DEA; oleyl alcohol; PEG-7M; PEG-14M; PEG-90M; stearamide DEA; stearamide MEA; stearyl alcohol; tragacanth gum; wheat starch; xanthan gum; and the likein the above list of thickeners, DEA is diethanolamine, and MEA is monoethanolamine. Thickeners (viscosity control agents) which may be used in effective amounts in nonaqueous systems include aluminum stearates; beeswax; candelilla wax; carnauba; ceresin; cetearyl alcohol; cetyl alcohol; cholesterol; hydrated silica; hydrogenated castor oil; hydrogenated cottonseed oil; hydrogenated soybean oil; hydrogenated tallow glyceride; hydrogenated vegetable oil; hydroxypropyl cellulose; lanolin alcohol; myristyl alcohol; octytdodecyl stearoyl sulfate; oleyl alcohol; ozokerite; microcystalline wax; paraffin, pentaerythrityl tetraoctanoate; polyacrylamide; polybutene; polyethylene; propylene glycol dicaprylate; propylene glycol dipelargonate; stearalkonium hectorite; stearyl alcohol; stearyl stearate; synthetic beeswax; trihydroxystearin; trilinolein; tristearin; zinc stearate; and the like.

Customary native and synthetic thickeners or gel formers in formulations are crosslinked polyacrylic acids and derivatives thereof, polysaccharides, such as xanthane gum or alginates, carboxymethylcellulose or hydroxycarboxymethylcellulose, hydrocolloids such as gum Arabic or montmorillonite minerals, such as bentonites or fatty alcohols, polyvinyl alcohol and polyvinlypyrrolidone.

Other ingredients which can be added or used in a cosmetic or pharmaceutical composition according to the invention in amounts effective for their intended use, include: biological additives to enhance performance or consumer appeal such as amino acids, proteins, vanilla, aloe extract, bioflavinoids, and the like; buffering agents, chelating agents such as EDTA; emulsion stabilizers; pH adjusters; opacifying agents; and propellants such as butane carbon clioxide, ethane, hydrochlorofluorocarbons 22 and 142b, hydrofluorocarbon 152a, isobutane, isopentane, nitrogen, nitrous oxide, pentane, propane, and the like.

Furthermore, the preparations according to the invention may also comprise compounds which have an antioxidative, free-radical scavenger, skin moisturizing or moisture-retaining, antierythematous,antiinflammatory or antiallergic action, in order to supplement or enhance their action. In particular, these compounds can be chosen from the group of vitamins, plant extracts, alpha- and beta-hydroxy acids, ceramides, antiinflammatory, antimicrobial or UV-filtering substances, and derivatives thereof and mixtures thereof. Advantageously, preparations according to the invention can also comprise substances which absorb UV radiation in the UV-B and/or UV-A region. The lipid phase is advantageously chosen from the group of substances of mineral oils, mineral waxes, branched and/or unbranched hydrocarbons and hydrocarbon waxes, triglycerides of saturated and/or unsaturated, branched and/or unbranched C.sub.8-C.sub.24-alkanecarboxylic acids; they can be chosen from synthetic, semisynthetic or natural oils, such as olive oil, palm oil, almond oil or mixtures; oils, fats or waxes, esters of saturated and/or unsaturated, branched and/or unbranched C.sub.3-C.sub.30-alkane carboxylic acids and saturated and/or unsaturated, branched and/or unbranched C.sub.3-C.sub.30-alcohols, from aromatic carboxylic acids and saturated and/or unsaturated, branched and/or unbranched C.sub.3-C.sub.30-alcohols, for example isopropyl myristate, isopropyl stearate, hexyldecyl stearate, oleyl oleate; and also synthetic, semisynthetic and natural mixtures of such esters, such as jojoba oil, alkyl benzoates or silicone oils, such as, for example, cyclomethicone, dimethylpolysiloxane, diethylpolysiloxane, octamethylcyclo-tetrasiloxane and mixtures thereof or dialkyl ethers.

The active ingredients according to the invention may, for example, be used in cosmetic compositions for the cleansing of the skin, such as bar soaps, toilet soaps, curd soaps, transparent soaps, luxury soaps, deodorizing soaps, cream soaps, baby soaps, skin protection soaps, abrasive soaps, syndets, liquid soaps, pasty soaps, soft soaps, washing pastes, liquid washing, showering and bath preparations, e.g. washing lotions, shower preparations, shower gels, foam baths, cream foam baths, oil baths, bath extracts, scrub preparations, in-situ products, shaving foams, shaving lotions, shaving creams. In addition, they are suitable for skin cosmetic preparations, such as W/O or O/W skin and body creams, day and night creams, light protection compositions, aftersun products, hand care products, face creams, multiple emulsions, gelees, microemulsions, liposome preparations, niosome preparations, antiwrinkle creams, face oils, lipogels, sportgels, moisturizing creams, bleaching creams, vitamin creams, skin lotions, care lotions, ampoules, aftershave lotions, preshaves, humectant lotions, tanning lotions, cellulite creams, depigmentation compositions, massage preparations, body powders, face tonics, deodorants, antiperspirants, nose strips, antiacne compositions, repellents and others.

In a preferred embodiment, a cosmetic composition comprises a daily care O/W formulation, which may contain, for example, the following ingredients in % in accordance with the International Nomenclature of Cosmetic Ingredients, INCl:

| | | |
|---|---|---|
| A | 1.7 | ceteareth-6, stearyl alcohol |
| | 0.7 | ceteareth-25 |
| | 2.0 | diethylamino hydroxybenzoyl hexyl benzoate |
| | 2.0 | PEG-14 dimethicone |
| | 3.6 | cetearyl alcohol |
| | 6.0 | ethylhexyl methoxycinnamate |
| | 2.0 | dibutyl adipate |
| B | 5.0 | glycerol |
| | 0.2 | disodium EDTA |
| | 1.0 | panthenol |
| | q.s. | preservative |
| | 67.8 | aqua dem. |
| C | 4.0 | caprylic/capric triglyceride, sodium acrylates copolymer |
| D | 0.2 | sodium ascorbyl phosphate |
| | 1.0 | tocopheryl acetate |
| | 0.2 | bisabolol |
| | 1.0 | caprylic/capric triglyceride, sodium ascorbate, tocopherol, retinol |
| | 1.0 | *Lactobacillus* spec. |
| E | q.s. | sodium hydroxide |

Phases A and B are separately heated to app. 80°C. Phase B is subsequently stirred into phase A and homogenized. Phase C is stirred into a combination of phases A and B and homogenized. The mixture is under agitation cooled down to app. 40°C; then phase D is added and the pH is adjusted with phase E to approx. 6.5. The solution is subsequently homogenized and cooled down to room temperature.

In a further preferred embodiment, a cosmetic composition comprises a protecting day cream O/W formulation, which may contain, for example, the following ingredients in % in accordance with the International Nomenclature of Cosmetic Ingredients, INCl:

| | | |
|---|---|---|
| A | 1.7 | ceteareth-6, stearyl alcohol |
| | 0.7 | ceteareth-25 |
| | 2.0 | diethylamino hydroxybenzoyl hexyl benzoate |
| | 2.0 | PEG-14 dimethicone |
| | 3.6 | cetearyl alcohol |
| | 6.0 | ethylhexyl methoxycinnamate |
| | 2.0 | dibutyl adipate |
| B | 5.0 | glycerol |
| | 0.2 | disodium EDTA |
| | 1.0 | panthenol |
| | q.s. | preservative |
| | 68.6 | aqua dem. |
| C | 4.0 | caprylic/capric triglyceride, sodium acrylates copolymer |
| D | 1.0 | sodium ascorbyl phosphate |
| | 1.0 | tocopheryl acetate |
| | 0.2 | bisabolol |
| | 1.0 | *Lactobacillus spec.* |
| E | q.s. | sodium hydroxide |

Phases A and B are separately heated to app. 80°C. Phase B is subsequently stirred into phase A and homogenized. Phase C is introduced into a combination of phases A and B and homogenized. The mixture is under agitation cooled down to app. 40°C; then phase D is added and the pH is adjusted with phase E to about 6.5. The solution is subsequently homogenized and cooled down to room temperature.

In a further preferred embodiment, a cosmetic composition comprises a skin cleanser O/W formulation, which may contain, for example, the following ingredients in % in accordance with the International Nomenclature of Cosmetic Ingredients, INCl:

| | | |
|---|---|---|
| A | 10.0 | cetearyl ethylhexanoate |
| | 10.0 | caprylic/capric triglyceride |
| | 1.5 | cyclopentasiloxane, cyclohexasilosane |
| | 2.0 | PEG-40 hydrogenated castor oil |
| B | 3.5 | caprylic/capric triglyceride, sodium acrylates copolymer |
| C | 1.0 | tocopheryl acetate |
| | 0.2 | bisabolol |
| | q.s. | preservative |
| | q.s. | perfume oil |
| D | 3.0 | polyquaternium-44 |
| | 0.5 | cocotrimonium methosulfate |
| | 0.5 | ceteareth-25 |
| | 2.0 | panthenol, propylene glycol |
| | 4.0 | propylene glycol |
| | 0.1 | disodium EDTA |
| | 1.0 | *Lactobacillus spec.* |
| | 60.7 | aqua dem. |

Initially, phase A is dissolved and phase B subsequently stirred into phase A. Subsequently, phase C is introduced into the combination of phases A and B. In a next step, phase D is dissolved and stirred into combined phases A, B and C. The mixture is homogenized and stirred for 15 min.

In a further preferred embodiment, a cosmetic composition comprises a daily care body spray formulation, which may contain, for example, the following ingredients in % in accordance with the International Nomenclature of Cosmetic Ingredients, INCl:

| | | |
|---|---|---|
| A | 3.0 | ethylhexyl methoxycinnamate |
| | 2.0 | diethylamino hydroxybenzoyl hexyl benzoate |
| | 1.0 | polyquaternium-44 |
| | 3.0 | propylene glycol |
| | 2.0 | panthenol, propylene glycol |
| | 1.0 | cyclopentasiloxane, cyclohexasiloxane |
| | 10.0 | octyldodecanol |
| | 0.5 | PVP |
| | 10.0 | caprylic/capric triglyceride |
| | 3.0 | C12-15 alkyl benzoate |
| | 3.0 | glycerol |
| | 1.0 | tocopheryl acetate |
| | 0.3 | bisabolol |
| | 1.0 | *Lactobacillus spec.* |
| | 59.2 | alcohol |

The components of phase A are weighed out and dissolved until clearness.

In a further preferred embodiment, a cosmetic composition comprises a skin gel, which may contain, for example, the following ingredients in % in accordance with the International Nomenclature of Cosmetic Ingredients, INCl:

| | | |
|---|---|---|
| | 3.6 | PEG-40 hydrogenated castor oil |
| | 15.0 | alcohol |
| | 0.1 | bisabolol |
| | 0.5 | tocopheryl acetate |
| | q.s. | perfume oil |
| B | 3.0 | panthenol |
| | 0.6 | carbomer |
| | 1.0 | *Lactobacillus spec.* |
| | 75.4 | aqua dem, |
| C | 0.8 | triethanolamine |

Initially, phase A is dissolved until clearness. Phase B is macerated and subsequently neutralized with phase C. In a next step, phase A is stirred into the homogenized phase B and the mixture is homogenized.

In yet a further preferred embodiment, a cosmetic composition comprises an after shave lotion, which may contain, for example, the following ingredients in % in accordance with the International Nomenclature of Cosmetic Ingredients, INCl:

| | | |
|---|---|---|
| A | 10.0 | cetearyl ethylhexanoate |
| | 5.0 | tocopheryl acetate |
| | 1.0 | bisabolol |
| | 0.1 | perfume oil |
| | 0.3 | acrylates/c10-30 alkyl acrylate crosspolymer |
| B | 15.0 | alcohol |
| | 1.0 | panthenol |
| | 3.0 | glycerol |
| | 1.0 | *Lactobacillus spec.* |
| | 0.1 | triethanolamine |
| | 63.5 | aqua dem. |

The component of phase A are mixed. In a next step, phase B is dissolved and introduced into phase A and subsequently homogenized.

The present invention also relates to the use of a microorganism according to the invention or of a mutant or inactive form thereof as described herein above for the preparation of a pharmaceutical composition for preventing or treating dermatitis, preferably atopic dermatitis, psoriasis, poison-ivy dermatitis, eczema herpeticum, kerion or scabies.

In another aspect the present invention relates to a method for the production of a composition comprising the step of formulating a microorganism of the invention or a mutant thereof or an inactive form as described herein above with a cosmetically and/or pharmaceutically carrier or excipient.

The present invention furthermore relates to a method of preventing or treating dermatitis, preferably atopic dermatitis, psoriasis, poison-ivy dermatitis, eczema herpeticum, kerion or scabies comprising the step of administering to a patient in need thereof a prophylactically or therapeutically effective amount of a composition according to the invention.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

It must be noted that as used herein and in the appended claims, the singular forms "a", "an", and "the", include plural referents unless the context clearly indicates otherwise. Thus, for example, reference to "a reagent" includes one or more of such different reagents, and reference to "the method" includes reference to equivalent steps and methods known to those of ordinary skill in the art that could be modified or substituted for the methods described herein.

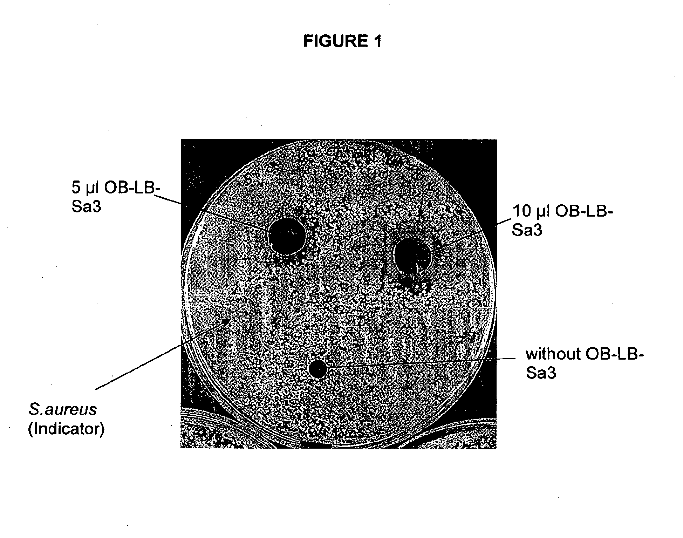

The invention is illustrated by Figures 1 to 7 as described in the following:
**Figure 1** shows the growth inhibition of *Staphylococcus aureus* in an *in vitro* hole/well plate assay (Example 1). The formation of a clear ring around the well indicates growth inhibition of the indicator strain Staphylococcus aureus.

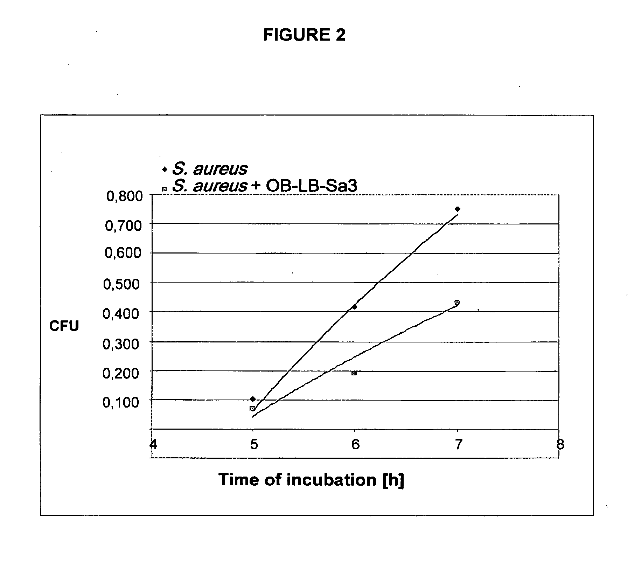

**Figure 2** shows growth inhibition of *Staphylococcus aureus* in an *in vitro* liquid assay (Example 2). Shown is the degree of inhibition which was quantified by counting the colony forming units of the indicator strain *Staphylococcus aureus* in comparison to a control without lactic acid bacteria.

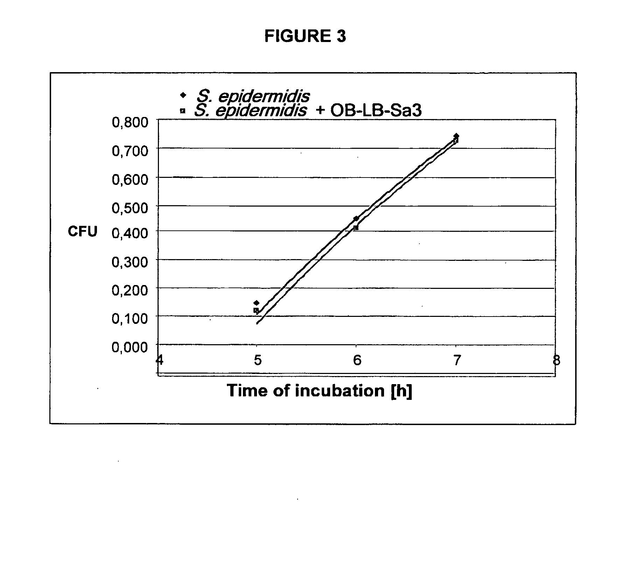

**Figure 3** shows the lack of growth inhibition of *Staphylococcus epidermidis* in an *in vitro* liquid assay (Example 3). Shown is the degree of inhibition which was quantified by counting the colony forming units of the indicator strain *Staphylococcus epidermidis* in comparison to a control without lactic acid bacteria.

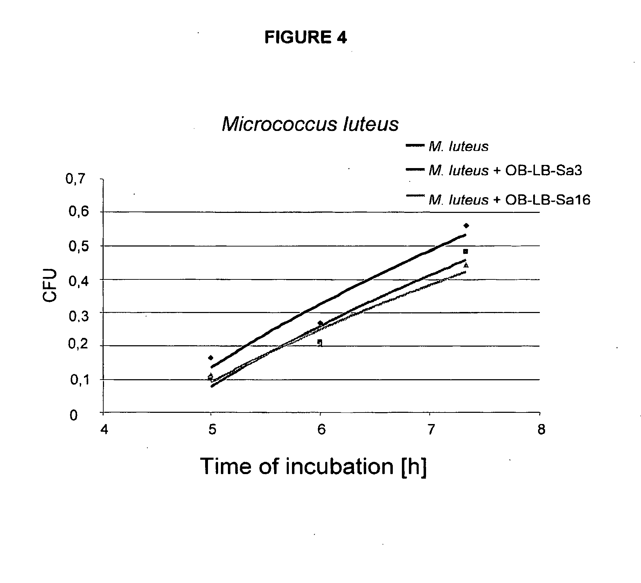

**Figure 4** shows the lack of growth inhibition of *Micrococcus luteus* in an in an *in vitro* liquid assay (Example 6). Shown is the degree of inhibition which was quantified by counting the colony forming units of the indicator strain *Micrococcus luteus* in comparison to a control without lactic acid bacteria.

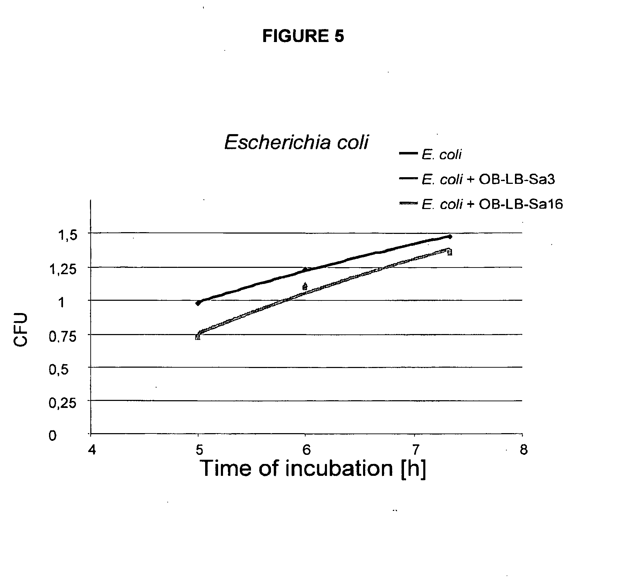

**Figure 5** shows the lack of growth inhibition of *Escherichia coli* in an in an *in vitro* liquid assay (Example 7). Shown is the degree of inhibition which was quantified by counting the colony forming units of the indicator strain *Escherichia coli* in comparison to a control without lactic acid bacteria.

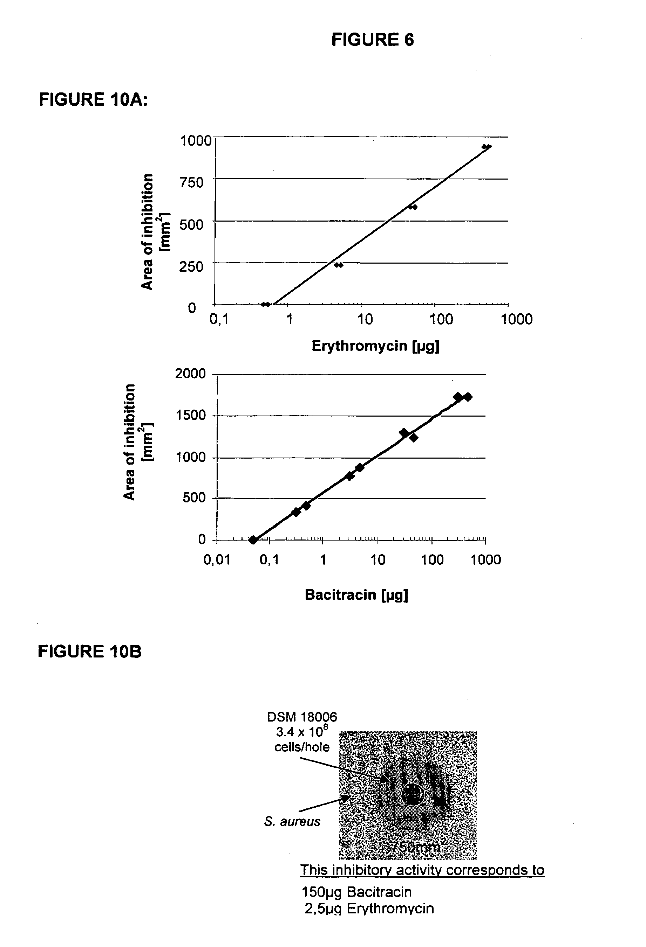

**Figure 6** shows the degree of growth inhibition of Staphylococcus aureus in an *in vitro* hole plate assay in comparison to bacitracin and erythromycin (Example 8). Bacitracin and erythromycin have been filled in precutted holes at different concentrations and the growth of Staphylococcus aureus has been observed. The corresponding calibration curves are shown in Figure 6A. The growth inhibition of S. aureus by a defined number of precultured Lactobacillus cells (DSM 18006) is shown in Figure 6B

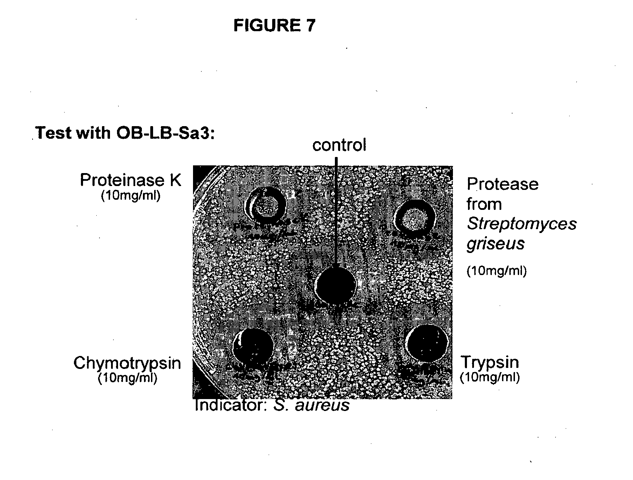

**Figure 7** shows the protease stability of Lactobacillus inhibitory substances (Example 9). Antimicrobial activity of Lactobacillus DSM 18006 has been characterized concerning the digestability by proteinase K, chymotrypsin, trypsin and protease from Streptomyces griseus.

The invention is further illustrated by the following Examples 1 to 9:

### Example 1

### Growth inhibition of S. aureus in an in vitro hole plate assay

Specific lactic acid bacteria have been identified, that are able to specifically inhibit the growth of *Staphylococcus aureus* on agar plates in an *in vitro* hole plate assay. To test this effect, pre cultured lactic acid bacteria have been filled into pre-cutted holes and a growth inhibition of the indicator strain S. *aureus* has been observed. Data are shown in Figure 1.

### Cultivation and preparation of lactobacilli:

Lactic acid bacteria were cultivated (OB-LB-Sa3; DSM 18006) from a -80°C freezing culture in 1 ml MRS broth in eppendorf tubes. Tubes were closed and cultivated for 2 days at 37°C. 10 µl of this pre culture was transferred to the main culture consisting of 7 ml MRS broth in falcon tubes. The culture was incubated for 2 days. After cultivation cells were harvested by centrifugation (15 min, 4000 x g). The cell pellet was washed two times with K/Na-buffer (each 1 ml). Cells were resuspended in 200 µl K/Na buffer.

### Cultivation and preparation of the indicator strain:

The indicator strain was *Staphylococcus aureus* (DSM346). 20 ml BHI broth in a shaking glass flask were inoculated with 15 µl of a 24 h pre culture. The indicator strain was cultivated for 24 h at 37°C. An aliquot was diluted to an optical density OD₅₉₅ₙₘ of 0.025 - 0.05 in BHI-broth and 800 µl spread on indicator plates (BHI). The agar was stamped using a cork borer. The holes were filled with 5 µl or 10 µl of the pre cultured lactic acid bacteria.

### Media and buffer:

| | | |
|---|---|---|
| BHI-Agar | Difco Agar 1.8%; 20 ml per plate | |
| BHI-Medium | Difco | |
| MRS-broth | Difco | |
| K/Na-buffer | according to Küster Thiel, pH 7.0, autoclaved | |
| | - 0.066 M Na₂HPO₄ x 2H₂O | 61.2 ml |
| | - 0.066 M KH₂PO₄ | 38.8 ml |

### Example 2

### Growth inhibition of S. aureus in an in vitro liquid assay

Specific lactic acid bacteria have been identified, that are able to specifically inhibit the growth of *Staphylococcus aureus* in liquid medium in an *in vitro* liquid assay. To test this effect, pre cultured lactic acid bacteria have been co-incubated with the indictator strain *S*. *aureus* in liquid cultivation medium, optimized for the growth of *Staphylococci*. The degree of inhibition was quantified by counting the colony forming units of the indicator strain in comparison to the control without lactic acid bacteria. Data are shown in Figure 2.

### Cultivation and preparation of lactobacilli:

Lactic acid bacteria were cultivated (OB-LB-Sa3; DSM 18006) from a -80°C freezing culture in 1 ml MRS broth in eppendorf tubes. Tubes was closed and cultivated for 2 days at 37°C. 10 µl of this pre culture was transferred to the main culture consisting of 7 ml MRS broth in falcon tubes. The culture was incubated for 2 days. After cultivation cells were harvested by centrifugation (15 min, 4000 x g). The cell pellet was washed two times with K/Na-buffer (each 1 ml). Cells were resuspended in 200 µl K/Na buffer with 250 mM glycerol and incubated for 17 h.

### Cultivation and preparation of the indicator strain:

The indicator strain was *Staphylococcus aureus* (DSM346). 10 ml BHI broth in a shaking glass flask were inoculated with 15 µl of a freezing culture for a 24 h pre culture. The culture was diluted with fresh BHI broth to a cell concentration of 2.5 x 10⁸ cells/ml.

### Liquid inhibition assay

For the liquid assay 5 µl of the freshly prepared lactic acid bacteria (out of 200 µl) and 10 µl of the pre cultured indicator strain *S*. *aureus* were inoculated for a co-cultivation in 10 ml of BHI broth. The culture was incubated for 7 h. Afterwards 100 µl of a 1:10000 dilution was spread on a BHI agar plate for quantification of the colony forming units. The plate was incubated for 24 h hours and the colony forming units were counted.

### Media and buffer:

| | | |
|---|---|---|
| BHI-Agar | Difco Agar 1,8%; 20 ml per plate | |
| BHI-Medium | Difco | |
| MRS-broth | Difco | |
| K/Na-buffer | according to Küster Thiel, pH 7.0, autoclaved | |
| | - 0.066 M Na₂HPO₄ x 2H₂O | 61.2 ml |
| | - 0.066 M KH₂PO₄ | 38.8 ml |

### Example 3

### No growth inhibition of Staphylococcus epidermidis an in vitro liquid assay

Using this assay it was possible to check whether selected lactic acid bacteria that were able to inhibit the growth of the pathogenic microorganism *Staphylococcus aureus* did not inhibit the major member of the commensal micro flora of the skin, *Staphylococcus epidermidis* in an *in vitro* liquid assay.

To test this effect, pre cultured lactic acid bacteria have been co-incubated with the indicator strain in a liquid culture. The degree of inhibition was quantified by counting the colony forming units of both indicator strains in comparison to the control without lactic acid bacteria. Data are shown in Figure 3.

### Cultivation and preparation of lactobacilli:

Lactic acid bacteria were cultivated (OB-LB-Sa3; DSM 18006) from a -80°C freezing culture in 1 ml MRS broth in eppendorf tubes. Tubes were closed and cultivated for 2 days at 37°C. 10 µl of this pre culture was transferred to the main culture consisting of 7 ml MRS broth in falcon tubes. The culture was incubated for 2 days. After cultivation cells were harvested by centrifugation (15 min, 4000 x g). The cell pellet was washed two times with K/Na-buffer (each 1 ml). Cells were resuspended in 200 µl K/Na buffer with 250 mM glycerol and incubated for 17 h.

### Cultivation and preparation of the indicator strain:

The indicator strain was *Staphylococcus epidermidis* (DSM20044). 20 ml BHI broth in a shaking glass flask was inoculated with 15 µl of a freezing culture for a 24 h pre culture.

### Liquid inhibition assay

For the liquid assay 5 µl of the freshly prepared lactic acid bacteria (out of 200 µl) and 10 µl of the pre cultured indicator strain *S*. *epidermidis* were inoculated for a co-cultivation in 10 ml of BHI broth. The culture was incubated for 7 h. Afterwards 100 µl of a 1:10000 dilution was spread on a BHI agar plate for quantification of the colony forming units. The plate was incubated for 24 h hours and the colony forming units were counted.

### Media and buffer:

| | | |
|---|---|---|
| BHI-Agar | Difco Agar 1.8%; 20 ml per plate | |
| BHI-Medium | Difco | |
| MRS-broth | Difco | |
| K/Na-buffer | according to Küster Thiel, pH 7.0, autoclaved | |
| | - 0.066 M Na₂HPO₄ x 2H₂O | 61.2 ml |
| | - 0.066 M KH₂PO₄ | 38.8 ml |

### Example 4

### Growth inhibition of Staphylococcus aureus in an in situ skin assay

Lactic acid bacteria have been identified that are able to inhibit the growth of *S*. *aureus* directly on the skin.

To test this effect, a culture of *Staphylococcus aureus* was diluted and directly applied to the skin and air dried. Afterwards an aliquot of the lactic acid bacterium was applied on this skin area. Thus the indicator strain *Staphylococcus aureus* was inhibited directly on the skin by the lactic acid bacterium. After incubation the staphylococci were transferred from the skin to an agar plate using in an adhesive tape. The agar plate was incubated at 37°C. A decreased colony count in comparison to the control without lactic acid bacteria indicates a growth inhibition of the indicator strain on the skin.

### Cultivation and preparation of lactobacilli:

Lactic acid bacteria were cultivated (OB-LB-Sa3; DSM 18006) from a -80°C freezing culture in 1 ml MRS broth in eppendorf tubes. Tubes were closed and cultivated for 2 days at 37°C. 10 µl of this pre culture were transferred to the main culture consisting of 7 ml MRS broth in falcon tubes. The culture was incubated for 2 days. After cultivation cells were harvested by centrifugation (15 min, 4000 x g). The cell pellet was washed two times with K/Na-buffer (each 1 ml). Cells are resuspended in 200 µl K/Na buffer.

### Cultivation and preparation of the indicator strain:

The indicator strain was *Staphylococcus aureus* (DSM346). 20 ml BHI broth in a shaking glass flask were inoculated with 15 µl of a 24 h pre culture. The indicator strain was cultivated for 24 h at 37°C.

### Media and buffer:

| | | |
|---|---|---|
| BHI-Agar | Difco Agar 1.8%; 20 ml per plate | |
| BHI-Medium | Difco | |
| MRS-broth | Difco | |
| K/Na-buffer | Küster Thiel, pH 7,0, autoclaved | |
| | - 0.066 M Na₂HPO₄ x 2H₂O | 61.2 ml |
| | - 0.066 M KH₂PO₄ | 38.8 ml |

### Application of S. aureus on the forearm:

400 µl of an 1:100 dilution of the prepared indicator strain *Staphylococcus aureus* was spread consistently on a defined skin area (10 cm x 3 cm) and air dried.

### Application of lactobacilli on the S. aureus inoculated skin area:

10 µl of prepared lactobacilli was applied to the *S*. *aureus* pre-inoculated skin area. The arm was incubated for six hours in a normal environment.

### Reisolation of microorganisms from the skin:

After 6 h the four upper skin layers were transferred to a BHI-agar plate using adhesive tape stripes. Thus the isolated skin bacteria were transferred to the agar plate. Agar plates were incubated for 24 h at 37°C.

### Example 5

### No growth inhibition of Staphylococcus epidermidis in an in situ skin assay

Lactic acid bacteria have been identified that inhibit the growth of *Staphylococcus aureus,* while the growth of *Staphylococcus epidermidis* is not affected directly on the skin.

Using this assay it was possible to check if the commensal microorganism *Staphylococcus epidermidis* of the healthy normal skin flora was not inhibited by lactic acid bacteria that are able to inhibit *Staphylococcus aureus.*

Therefore the indicator strain *Staphylococcus epidermidis* was applied highly diluted to the skin in the same manner as *Staphylococcus aureus.* Again the inhibiting activity of lactic acid bacteria was tested. An inhibition of *Staphylococcus epidermidis* has not been observed with the described lactic acid bacteria.

### Cultivation and preparation of lactobacilli:

Lactic acid bacteria were cultivated (OB-LB-Sa3; DSM 18006) from a -80°C freezing culture in 1 ml MRS broth in eppendorf tubes. Tubes were closed and cultivated for 2 days at 37°C. 10 µl of this pre culture was transferred to the main culture consisting of 7 ml MRS broth in falcon tubes. The culture was incubated for 2 days. After cultivation cells were harvested by centrifugation (15 min, 4000 x g). The cell pellet was washed two times with K/Na-buffer (each 1 ml). Cells were resuspended in 200 µl K/Na buffer.

### Cultivation and preparation of the indicator strain:

The indicator strain was *Staphylococcus epidermidis* (DSM20044). 20 ml BHI broth in a shaking glass flask were inoculated with 15 µl of a 24 h pre culture. The indicator strain was cultivated for 24 h at 37°C.

### Media and buffer:

| | | |
|---|---|---|
| BHI-Agar | Difco Agar 1.8%; 20 ml per plate | |
| BHI-Medium | Difco | |
| MRS-broth | Difco | |
| K/Na-buffer | Küster Thiel, pH 7.0, autoclaved | |
| | - 0.066 M Na₂HPO₄ x 2H₂O | 61.2 ml |
| | - 0.066 M KH₂PO₄ | 38.8 ml |

### Application of Staphylococcus epidermidis on the forearm:

400 µl of a 1:100 dilution of the prepared indicator strain *Staphylococcus epidermidis* was spread consistently on a defined skin area (10 cm x 3 cm) and air dried.

### Application of lactobacilli on the S. epidermidis inoculated skin area:

10 µl of prepared lactobacilli were applied to the *S*. *epidermidis* pre-inoculated skin area. The arm was incubated for six hours in a normal environment.

### Reisolation of microorganisms from the skin:

After 6 h the four upper skin layers was transferred to a BHI-agar plate using adhesive tape stripes. Thus the isolated skin bacteria are transferred to the agar plate. Agar plates are incubated for 24 h at 37°C.

### Example 6

### No growth inhibition of Micrococcus luteus in the in-vitro- liquid assay

The selected lactic acid bacteria that are able to inhibit the growth of the pathogenic microorganism *Staphylococcus aureus* do not inhibit the relevant member of the commensal micro flora of the skin, *Micrococcus luteus* in an *in vitro* liquid assay.

To test this effect, pre cultured lactic acid bacteria have been co-incubated with the indictator strain in a liquid culture. The degree of inhibition was quantified by counting the colony forming units of both indicator strains in comparison to the control without lactic acid bacteria. Data are shown in Figure 4.

### Cultivation and preparation of lactobacilli:

Lactic acid bacteria were cultivated (OB-LB-Sa3; DSM 18006 and OB-LB-Sa16; DSM 18007) from a -80°C freezing culture in 1 ml MRS broth in eppendorf tubes. Tubes were closed and cultivated for 2 days at 37°C. 10 µl of this pre culture was transferred to the main culture consisting of 7 ml MRS broth in falcon tubes. The culture was incubated for 2 days. After cultivation cells were harvested by centrifugation (15 min, 4000 x g). The cell pellet was washed two times with K/Na-buffer (each 1 ml). Cells were resuspended in 200 µl K/Na buffer with 250 mM glycerol and incubated for 17 h.

### Cultivation and preparation of the indicator strain:

The indicator strain was *Micrococcus luteus.* 20 ml BHI broth in a shaking glass flask was inoculated with 15 µl of a freezing culture for a 24 h pre culture.

### Liquid inhibition assay:

For the liquid assay 5 µl of the freshly prepared lactic acid bacteria (out of 200 µl) and 10 µl of the pre cultured indicator strain *M. luteus* were inoculated for a co-cultivation in 10 ml of BHI broth. The culture was incubated for 7 h. Afterwards 100 µl of a 1:1000 dilution was spread on a BHI agar plate for quantification of the colony forming units. The plate was incubated for 24 h and the colony forming units were counted.

### Media and buffer:

| | | |
|---|---|---|
| BHI-Agar | Difco Agar 1.8%; 20 ml per plate | |
| BHI-Medium | Difco | |
| MRS-broth | Difco | |
| K/Na-buffer | according to Küster Thiel, pH 7.0, autoclaved | |
| | - 0.066 M Na₂HPO₄ x 2H₂O | 61.2 ml |
| | - 0.066 M KH₂PO₄ | 38.8 ml |

### Example 7

### No growth inhibition of Escherichia coli in the in-vitro- liquid assay

The selected lactic acid bacteria that are able to inhibit the growth of the pathogenic microorganism *Staphylococcus aureus* do not inhibit other human relevant microorganisms, e.g *Escherichia coli* in an *in vitro* liquid assay.

To test this effect, pre cultured lactic acid bacteria have been co-incubated with the indicator strain in liquid culture. The degree of inhibition was quantified by counting the colony forming units of both indicator strains in comparison to the control without lactic acid bacteria. Data are shown in Figure 5.

### Cultivation and preparation of lactobacilli:

Lactic acid bacteria were cultivated (OB-LB-Sa3; DSM 18006 and OB-LB-Sa16; DSM 18007) from a -80°C freezing culture in 1 ml MRS broth in eppendorf tubes. Tubes were closed and cultivated for 2 days at 37°C. 10 µl of this pre culture was transferred to the main culture consisting of 7 ml MRS broth in falcon tubes. The culture was incubated for 2 days. After cultivation cells were harvested by centrifugation (15 min, 4000 x g). The cell pellet was washed two times with K/Na-buffer (each 1 ml). Cells were resuspended in 200 µl K/Na buffer with 250 mM glycerol and incubated for 17 h.

### Cultivation and preparation of the indicator strain:

The indicator strain was *Escherichia coli.* 20 ml BHI broth in a shaking glass flask was inoculated with 15 µl of a freezing culture for a 24 h pre culture.

### Liquid inhibition assay:

For the liquid assay 5 µl of the freshly prepared lactic acid bacteria (out of 200 µl) and 10 µl of the pre cultured indicator strain *E*. *coli* were inoculated for a co-cultivation in 10 ml of BHI broth. The culture was incubated for 7 h. Afterwards 100 µl of a 1:1000 dilution was spread on a BHI agar plate for quantification of the colony forming units. The plate was incubated for 24 h and the colony forming units were counted.

### Media and buffer:

| | | |
|---|---|---|
| BHI-Agar | Difco Agar 1.8%; 20 ml per plate | |
| BHI-Medium | Difco | |
| MRS-broth | Difco | |
| K/Na-buffer | according to Küster Thiel, pH 7.0, autoclaved | |
| | - 0.066 M Na₂HPO₄ x 2H₂O | 61.2 ml |
| | - 0.066 M KH₂PO₄ | 38.8 ml |

### Example 8

### Degree of growth inhibition of S. aureus in an in-vitro- hole plate assay in comparison to bacitracin and erythromycin

### Specific lactic acid bacteria have been identified, that are able to specifically inhibit the growth of Staphylococcus aureus on agar plates in an in-vitro- hole plate assay. This effect has been compared to commercial antibiotic cream preparations of bacitracin and erythromycin. To compare this effect, both antibiotics have been filled into pre-cutted holes at different concentrations and a growth inhibition of the indictator strain S. aureus has been observed (calibration curves in Figure 6A). The diameter of the inhibition zones has been measured and the area of inhibition has been calculated thereof. Afterwards this area has been correlated to the growth inhibition of S. aureus by defined numbers of precultured Lactobacillus cells of strain OB-LB-Sa3 (DSM 18006) (see Figure 6B).

### Cultivation and preparation of lactobacilli:

Lactic acid bacteria were cultivated (OB-LB-Sa3; DSM 18006) from a -80°C freezing culture in 1 ml MRS broth in eppendorf tubes. Tubes were closed and cultivated for 2 days at 37°C. 10 µl of this pre culture was transferred to the main culture consisting of 7 ml MRS broth in falcon tubes. The culture was incubated for 2 days. After cultivation cells were harvested by centrifugation (15 min, 4000 x g). The cell pellet was washed two times with K/Na-buffer (each 1 ml). Cells were resuspended in 200 µl K/Na buffer.

### Cultivation and preparation of the indicator strain:

The indicator strain was *Staphylococcus aureus* (DSM346). 20 ml BHI broth in a shaking glass flask were inoculated with 15 µl of a 24 h pre culture. The indicator strain was cultivated for 24 h at 37°C. An aliquot was diluted to an optical density OD₅₉₅ₙₘ of 0.025 - 0.05 in BHI-broth and 800 µl spread on indicator plates (BHI). The agar was stamped using a cork borer. The holes were filled with 5 µl or 10 µl of the pre cultured lactic acid bacteria or corresponding volumes of commercial antibiotic preparations.

### Media and buffer:

| | | |
|---|---|---|
| BHI-Agar | Difco Agar 1.8%; 20 ml per plate | |
| BHI-Medium | Difco | |
| MRS-broth | Difco | |
| K/Na-buffer | according to Küster Thiel, pH 7.0, autoclaved | |
| | - 0.066 M Na₂HPO₄ x 2H₂O | 61.2 ml |
| | - 0.066 M KH₂PO₄ | 38.8 ml |

### Example 9

### Protease stability of Lactobacillus inhibitory substance

Specific lactic acid bacteria have been identified, that are able to specifically inhibit the growth of *Staphylococcus aureus* on agar plates in an *in-vitro-* hole plate assay. The antimicrobial activity of selected lactobacilli has been characterized concerning digestibility by proteinase K, proteas from Streptomyces griseus, chymotrypsin and trypsin. Cell free preparations of *Lactobacillus* supernatants have been prepared and incubated with different proteases for 1 h at 37°C. Afterwards these preparations have been tested for their ability to inhibit the growth of the indicator strain S. *aureus.* The diameter of the inhibition zones has been measured and the area of inhibition has been calculated thereof (see Figure 7).

### Cultivation and preparation of lactobacilli:

Lactic acid bacteria were cultivated (OB-LB-Sa3; DSM 18006) from a -80°C freezing culture in 7 ml MRS broth in falcon tubes. Tubes were closed and cultivated for 2 days at 37°C. 7 ml of this pre culture was transferred to the main culture consisting of 40 ml MRS broth in flasks. The culture was incubated for 2 days. After cultivation cells were harvested by centrifugation (15 min, 4000 x g). The cell pellet was washed two times with K/Na-buffer (each 2 ml). Cells were resuspended in 10 ml BHI medium and incubated for 6 h at 37°C. Cells were harvested by centrifugation (15 min, 4000 x g) and the supernatant was used for protease incubation. In detail, 150 µl of the supernatant was incubated with 15 µl of a 10 mg/ml protease solution at 37°C.

### Cultivation and preparation of the indicator strain:

The indicator strain was *Staphylococcus aureus* (DSM346). 20 ml BHI broth in a shaking glass flask were inoculated with 15 µl of a 24 h pre culture. The indicator strain was cultivated for 24 h at 37°C. An aliquot was diluted to an optical density OD₅₉₅ₙₘ of 0.025 - 0.05 in BHI-broth and 800 µl spread on indicator plates (BHI). The agar was stamped using a cork borer. The holes were filled with 5 µl or 10 µl of the pre cultured cells and was incubated with 15 µl of a 10 mg/ml protease solution at 37°C for 1 h. Afterwards 5 µl or 10 µl of the protease treated lactobacillus supernatant was used for the inhibition assay

### Media and buffer:

| | | |
|---|---|---|
| BHI-Agar | Difco Agar 1.8%; 20 ml per plate | |
| BHI-Medium | Difco | |
| MRS-broth | Difco | |
| K/Na-buffer | according to Küster Thiel, pH 7.0, autoclaved | |
| | - 0.066 M Na₂HPO₄ x 2H₂O | 61.2 ml |
| | - 0.066 M KH₂PO₄ | 38.8 ml |

### Cited References

Aly, R., Maibach, HI., Shinefield, HR., Strauss, WG. (1972): Survival of pathogenic microorganisms on human skin. J Invest Dermatol. 58(4): 205-210.
Bisno, AL. (1984): Cutaneous infections: microbiologic and epidemiologic considerations. Am J Med. 76(5A): 172-179.
Brook, I. (2000): The effects of amoxicillin therapy on skin flora in infants. Pediatr Dermatol. 17(5): 360-363.
Elek, SD. (1956): Experimental staphylococcal infections in the skin of man. Ann. NY Acad Sci. 65: 85-90.
Feingold, DS. (1985): Cutaneous microbial flora. Cutis. 36(5A): 1.
Gfatter, R., Hackl, P., Braun, F. (1997): Effects of soap and detergents on skin surface pH, stratum corneum hydration and fat content in infants. Dermatology. 195(3): 258-262.
Gibbons, RJ., Houte, JV. (1975): Bacterial adherence in oral microbial ecology. Annu Rev Microbiol. 1975;29: 19-44.
Hurst, V. (1959): Transmission of hospital staphylococci among newborn infants. Pediatrics 25: 204-214.
Imokawa, G., Akasaki, S., Hattori, M., Yoshizuka, N. (1986): Selective recovery of deranged water-holding properties by stratum corneum lipids. J Invest Dermatol. 87(6): 758-761.
Korting, HC. (1992): Einfluß des pH-Wertes auf das Wachstum von Staphylococcus epidermidis, Staphylococcus aureus und Propionibacterium acnes in kontinuierlicher Kultur. Zbl. Hyg. 193: 78-90.
Korting, HC., Hübner, K., Greiner, K., Hamm, G., Braun-Falco, O. (1990): Unterschiede des Hautoberflächen-pH-Wertes und der bakteriellen Mikroflora durch Langzeit-Anwendung synthetische Detergenz-Zubereitungen mit pH 5,5 und pH 7,0 in Acta Derm Venereol. 70: 429-457.
Larson, E. (2001): Hygiene of the skin: when is clean too clean? Emerg Infect Dis. 7(2): 225-230.
Leyden, JJ., McGinley, KJ., Nordstrom, KM., Webster, GF. (1987): Skin microflora. J Invest Dermatol. 88(3): 65-72.
Lukas, A. (1990): Beeinflußbarkeit des Wachstums wichtiger Bakterien der Residentflora in-vitro durch den pH-Wert. In: O. Braun-Falco, HC. Korting (Hrsg.): Hautreinigung mit Syndets, 104-112.
Milyani, RM., Selwyn, S. (1978): Quantitative studies on competitive activities of skin bacteria growing on solid media. J Med Microbiol. 11 (4): 379-386.
Ohnishi, Y., Okino, N., Ito, M., Imayama, S. (1999): Ceramidase activity in bacterial skin flora as a possible cause of ceramide deficiency in atopic dermatitis. Clin Diagn Lab Immunol. 6(1): 101-104.
Roth, RR., James, WD. (1988): Microbial ecology of the skin. Annu Rev Microbiol. 42: 441-464.
Selwyn, S., Ellis, H. (1972): Skin bacteria and skin disinfection reconsidered. Br Med J. 1(793): 136-140.
Sullivan, A., Edlund, C., Nord, CE. (2001): Effect of antimicrobial agents on the ecological balance of human micro flora. Lancet Infect Dis. 1(2): 101-114.
Yosipovitch, G., Maibach, HI. (1996): Skin surface pH: A protective acid mantle in Cosmetics Toiletries magazine 111 (12): 101

## Claims

1. A microorganism selected from the group consisting of Lactobacillus buchneri having DSMZ accession number DSM 18007 and Lactobacillus delbrulckii having DSMZ accession number DSM 18006, or a mutant thereof, wherein said microorganism has or the mutant retains
(i) the ability to inhibit the growth of microorganisms selected from the group consisting of Staphylococcus aureus, Streptococcus pyogenes, gram-negative bacilli, Candida albicans and Malassezia furfur, and
(ii) the ability not to inhibit the growth of Staphylococcus epidermidis,
wherein inhibition is determined by a decrease of growth of at least 5% of the respective microorganism in an in-vitro hole plate assay.

2. An inactive form of the microorganism of claim 1, which is thermally inactivated or lyophilized, and
(i) able to inhibit the growth of microorganisms selected from the group consisting of Staphylococcus aureus, Streptococcus pyogenes, gram-negative bacilli, Candida albicans and Malassezia furfur, and which
(ii) does not inhibit the growth of Staphylococcus epidermidis,
wherein inhibition is determined by a decrease of growth of at least 5% of the respective microorganism in an in-vitro hole plate assay.

3. A composition comprising a microorganism of claim 1 or an inactive form of claim 2.

4. The composition of claim 3, which is
(a) a cosmetic composition optionally comprising a cosmetically acceptable carrier or exipient, and/or
(b) a pharmaceutical composition optionally comprising a pharmaceutically acceptable carrier or exipient.

5. Use of a microorganism of claim 1 or of an inactive form of claim 2 for the preparation of a cosmetic composition for protecting skin against pathogenic bacteria.

6. A microorganism of claim 1 or of an inactive form of claim 2 for use in a method for the prophylaxis or treatment of dermatitis.

7. The microorganism for use of claim 6, wherein the dermatitis is atopic dermatitis, psoriasis, poison-ivy dermatitis, eczema herpeticum, kerion or scabies.

8. A method for the production of a composition comprising the step of formulating a microorganism according to claim 1 or of an inactive form of claim 2 with a cosmetically or pharmaceutically acceptable carrier or excipient.

## Patentansprüche

1. Mikroorganismus, der aus der Gruppe ausgewählt ist, die aus Lactobacillus buchneri mit der DSM-Zugangsnummer DSM 18007 und Lactobacillus delbrückii mit der DSM-Zugangsnummer DSM 18006 oder einem Mutanten davon besteht, wobei der Mikroorganismus folgendes aufweist oder der Mutant folgendes bewahrt:
(i) die Fähigkeit, das Wachstum von Mikroorganismen, die aus der Gruppe ausgewählt sind, die aus Staphylococcus aureus, Streptococcus pyrogenes, gram-negativen Bazillen, Candida albicans und Malassezia furfur besteht, zu inhibieren, und
(ii)die Fähigkeit, das Wachstum von Staphylococcus epidermis nicht zu inhibieren,
wobei die Inhibition durch eine Abnahme des Wachstums des betreffenden Mikroorganismus um wenigstens 5 % in einem in vitro-Lochplattentest bestimmt wird.

2. Inaktive Form des Mikroorganismus nach Anspruch 1, der thermisch inaktiviert oder lyophilisiert ist, und
(i) der in der Lage ist, das Wachstum von Mikroorganismen, die aus der Gruppe ausgewählt sind, die aus Staphylococcus aureus, Streptococcus pyrogenes, gram-negativen Bazillen, Candida albicans und Malassezia furfur besteht, zu inhibieren, und
(ii)der das Wachstum von Staphylococcus epidermis nicht inhibiert,
wobei die Inhibition durch eine Abnahme des Wachstums des betreffenden Mikroorganismus um wenigstens 5 % in einem in vitro-Lochplattentest bestimmt wird.

3. Zusammensetzung, die einen Mikroorganismus nach Anspruch 1 oder eine inaktive Form nach Anspruch 2 umfasst.

4. Zusammensetzung nach Anspruch 3, welche
(a) eine kosmetische Zusammensetzung optional mit einem kosmetisch akzeptablen Träger oder Hilfsstoff und/oder
(b) eine pharmazeutische Zusammensetzung optional mit einem pharmazeutisch akzeptablen Träger oder Hilfsstoff ist.

5. Verwendung eines Mikroorganismus nach Anspruch 1 oder einer inaktiven Form nach Anspruch 2 für die Herstellung einer kosmetischen Zusammensetzung zum Schutz der Haut vor pathogenen Bakterien.

6. Mikroorganismus nach Anspruch 1 oder inaktive Form nach Anspruch 2 zur Verwendung bei einem Verfahren für die Prophylaxe oder Behandlung von Dermatitis.

7. Mikroorganismus zur Verwendung nach Anspruch 6, wobei die Dermatitis atopische Dermatitis, Psoriasis, Giftefeu-Dermatitis, Eczema herpeticatum, Kerion oder Krätze ist.

8. Verfahren zur Herstellung einer Zusammensetzung, umfassend den Schritt des Formulierens eines Mikroorganismus nach Anspruch 1 oder einer inaktiven Form nach Anspruch 2 mit einem kosmetisch oder pharmazeutisch akzeptablen Träger oder Hilfsstoff.

## Revendications

1. Microorganisme choisi parmi le groupe constitué de Lactobacillus buchneri ayant le numéro d'accès DSM 18007 de la DSMZ et Lactobacillus delbruckii ayant le numéro d'accès DSM 18006 de la DSMZ ou d'un mutant de ceux-ci, le microorganisme ayant ou le mutant retenant:
(i) la capacité d'inhiber la croissance de microorganismes choisis parmi le groupe constitué de Staphylococcus aureus, Streptococcus pyrogenes, bacilles gram négatif, Candida albicans et Malassezia furfur, et
(ii)la capacité de ne pas inhiber la croissance de Staphylococcus epidermis,
l'inhibition étant déterminée par une diminution de la croissance du microorganisme respectif par au moins 5 % dans un essai in vitro d'une plaque à puits.

2. Forme inactive du microorganisme selon la revendication 1, qui est inactivé thermiquement ou lyophilisée, et
(i) qui est capable d'inhiber la croissance de microorganismes choisis parmi le groupe constitué de Staphylococcus aureus, Streptococcus pyrogenes, bacilles gram négatif, Candida albicans et Malassezia furfur, et
(ii) qui n'inhibe pas la croissance de Staphylococcus epidermis,
dans laquelle l'inhibition est déterminée par une diminution de la croissance du microorganisme respectif par au moins 5 % dans un essai in vitro d'une plaque à puits.

3. Composition, qui comprend un microorganisme selon la revendication 1 ou une forme inactive selon la revendication 2.

4. Composition selon la revendication 3, qui est
(a) une composition cosmétique optionnellement avec un support ou un excipient cosmétiquement acceptable et/ou
(b) une composition pharmaceutique optionnellement avec un support ou un excipient pharmaceutiquement acceptable.

5. Utilisation d'un microorganisme selon la revendication 1 ou d'une forme inactive selon la revendication 2 pour la préparation d'une composition cosmétique pour la protection de la peau de bactéries pathogènes.

6. Microorganisme selon la revendication 1 ou forme inactive selon la revendication 2 pour l'utilisation dans un procédé de prophylaxie ou traitement de la dermatite.

7. Microorganisme pour l'utilisation selon la revendication 6, dans lequel la dermatite est la dermatite atopique, le psoriasis, la dermatite du
lierre vénéneux, l'eczéma herpeticatum, le kérion ou la gale.

8. Procédé de préparation d'une composition, comprenant l'étape de formuler un microorganisme selon la revendication 1 ou une forme inactive selon la revendication 2 avec un support ou un excipient cosmétiquement ou pharmaceutiquement acceptable.
